# EUROPEAN PATENT APPLICATION

(11) **EP 4 790 049 A1**
(43) Date of publication of application: **12.08.2026**
(21) Application number: 24874087.0
(22) Date of filing: 30.09.2024
(51) Int. Cl.: C07D 207/16, A61K 31/401, A61K 31/40, A61P 25/28, A61P 25/24, A61P 25/16, A61P 25/00, A61P 25/26

(54) **BIPHENYL TETRAHYDROPYRROLE COMPOUND, PREPARATION METHOD THEREFOR AND USE THEREOF**

(30) Priority: 07.10.2023 CN 202311280724
(71) Applicant: Hainan Yinrui Bio-Pharm Co., Ltd., Haikou, Hainan 570000 (CN)
(72) Inventor: QIN, Bingjie, Haikou, Hainan 570000 (CN); WANG, Dajing, Haikou, Hainan 570000 (CN)
(74) Representative: Kador & Partner Part mbB
(86) International application number: PCT/CN2024/122874
(87) International publication number: WO 2025/073270

(57) **Abstract**

The present invention discloses a biphenyltetrahydropyrrole compound of Formula I, a preparation method therefor, and a use thereof as a monoamine oxidase B inhibitor. The compound of the present invention exhibits selective activity against MAO-B and has almost no activity against MAO-A, effectively reducing side effects caused by acting on monoamine oxidase A.

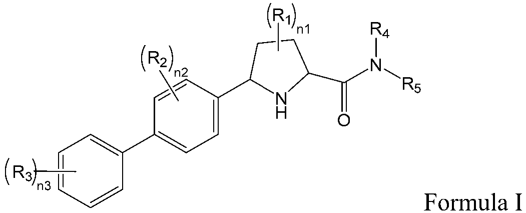

## Description

### Technical Field

The present invention belongs to the field of medicinal chemistry, and specifically relates to a biphenyltetrahydropyrrole compound or an isotopically labeled compound thereof, or an optical isomer, geometric isomer, tautomer or isomer mixture thereof, or a pharmaceutically acceptable salt thereof, or a prodrug thereof, or a metabolite thereof, and a pharmaceutical composition thereof. The compound of Formula I or the pharmaceutical composition can serve as a monoamine oxidase B inhibitor for treating or preventing a disease or condition associated with monoamine oxidase B.

### Background Art

Monoamine oxidase (MAO) is located on the outer mitochondrial membrane and primarily catalyzes oxidative deamination reactions. MAO is divided into two subtypes, MAO-A and MAO-B, consisting of 527 and 520 amino acid residues respectively, with 72.6% amino acid sequence identity. Due to differences in their three-dimensional spatial structures, MAO-A and MAO-B exhibit a certain degree of substrate selectivity. MAO-A primarily metabolizes serotonin (5-HT), norepinephrine (NE), etc., while MAO-B has higher affinity for benzylamine and β-phenethylamine. Dopamine (DA) can serve as a substrate for both MAO-A and MAO-B. MAO is widely distributed in the central and peripheral nervous systems. MAO-A is present in higher concentrations in noradrenergic neurons projecting from the locus coeruleus, whereas MAO-B is found in higher levels in serotonergic and histaminergic neurons of the raphe nuclei and posterior hypothalamus. In dopaminergic neurons of the substantia nigra, MAO-A is predominantly expressed, while MAO-B is mainly expressed in glial cells of the substantia nigra. Quantitative analysis reveals that MAO-B levels in the substantia nigra are three times those of MAO-A. The tissue-specific distribution of the two MAO subtypes determines their substrate specificity. In the human brain, MAO-B activity accounts for more than 80% of total MAO activity; therefore, it is considered that striatal DA in the brain is primarily inactivated via deamination by MAO-B. Nonselective MAO inhibitors have been used clinically for the treatment of Depression since the 1950s, but their use was discontinued due to severe "cheese effects." Nonselective MAO inhibitors not only increase the levels of catecholamine neurotransmitters in the central nervous system but also indirectly enhance the sympathomimetic effects of peripheral monoamine neurotransmitters, such as the hypertensive crisis induced by tyramine (an MAO-A substrate). Further research on MAO has revealed that MAO-B activity is significantly elevated in the brains of elderly individuals and patients with neurodegenerative diseases. A possible reason is that aging and neuronal damage lead to an increase in microglia, which in turn causes an increase in MAO-B activity. Consequently, selective MAO-B inhibitors have gradually been used in the treatment of neurodegenerative diseases such as PD, including Selegiline, Rasagiline, and Safinamide. Selegiline and Rasagiline are two marketed selective, irreversible MAO-B inhibitors. When combined with levodopa, they significantly increase striatal DA levels. The UKPDRG trial found that patients taking selegiline 10 mg/day experienced increased mortality, possibly due to the drug's poor selectivity or cardiovascular regulatory impairment caused by their metabolite, amphetamine. Rasagiline has high selectivity for MAO-B. However, due to its irreversible inhibition and the fact that MAO regeneration in the human body requires nearly 30 days, there remains a risk associated with its use.

Safinamide is a third-generation monoamine oxidase inhibitor developed by Newron, an Italian company. Compared to the previous two generations (Selegiline, Rasagiline), it features high selectivity and reversibility. Safinamide exhibits significantly greater selectivity for MAO-B than Selegiline and Rasagiline, thereby avoiding the side effects associated with the poor selectivity of Selegiline and Rasagiline during use. Furthermore, this inhibition is reversible, with normal physiological function restored 8 hours after discontinuation. Safinamide not only inhibits MAO-B activity but also increases striatal dopamine content by inhibiting dopamine reuptake. It also inhibits excessive glutamate release by blocking sodium channels and modulating calcium channels, conferring neuroprotective effects. However, due to characteristics such as poor pharmacokinetics, its effective dose is relatively high. Therefore, developing high-efficacy, high-selectivity, reversible MAO-B inhibitors remains an important task in the PD field.

In summary, MAO-B, as an important enzyme, plays a key role in neurotransmitter metabolism, disease occurrence, and treatment. Further research remains necessary for a better understanding and application of the role of MAO-B, which will contribute to a better understanding and treatment of diseases related to MAO-B.

### Summary of the Invention

The present invention provides a novel biphenyltetrahydropyrrole compound, or an isotopically labeled compound thereof, or an optical isomer, geometric isomer, tautomer or isomer mixture thereof, or a pharmaceutically acceptable salt thereof, or a prodrug thereof, or a metabolite thereof, which have not been reported previously. The compounds can serve as a monoamine oxidase B (MAO-B) inhibitor. The compounds exhibit selective activity against MAO-B and have almost no activity against MAO-A.

According to one aspect of the present invention, an object of the present invention is to provide a biphenyltetrahydropyrrole compound of Formula I, or an isotopically labeled compound thereof, or an optical isomer, geometric isomer, tautomer or isomer mixture thereof, or a pharmaceutically acceptable salt thereof, or a prodrug thereof, or a metabolite thereof: 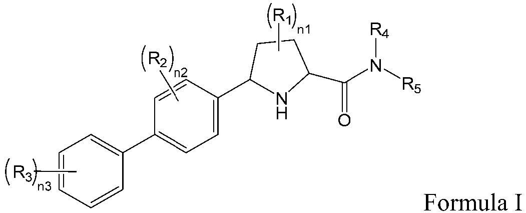 wherein,
R₁ is selected from the group consisting of hydrogen, deuterium, halogen, cyano, hydroxy, amino, amido, sulfonyl, C₁-C₆ alkyl, C₃-C₆ cycloalkyl, C₁-C₆ alkoxy, and C₃-C₆ cycloalkoxy;
R₂ is selected from the group consisting of hydrogen, deuterium, halogen, cyano, hydroxy, amino, amido, sulfonyl, C₁-C₆ alkyl, C₃-C₆ cycloalkyl, C₁-C₆ alkoxy, and C₃-C₆ cycloalkoxy;
R₃ is selected from the group consisting of hydrogen, deuterium, halogen, cyano, hydroxy, nitro, carboxy, amino, amido, sulfonyl, C₁-C₆ alkyl, C₁-C₆ haloalkyl, C₃-C₆ cycloalkyl, C₁-C₆ alkoxy, C₁-C₆ haloalkoxy, C₃-C₆ cycloalkoxy, -C(=O)OC₁-C₆ alkyl, and -C(=O)ONH₂;
R₄ and R₅ are each independently selected from the group consisting of hydrogen, halogen, cyano, amido, sulfonyl, C₁-C₆ alkyl, C₃-C₆ cycloalkyl, C₁-C₆ alkoxy, and C₃-C₆ cycloalkoxy;
n1 is an integer selected from 0 to 2.
n2 is an integer selected from 0 to 4.
n3 is an integer selected from 0 to 5.

Preferably, R₁ is selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy.

Preferably, R₁ is selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexyloxy.

Preferably, R₁ is selected from the group consisting of hydrogen, deuterium, halogen, cyano, and hydroxy.

Preferably, R₂ is selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy.

Preferably, R₂ is selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexyloxy.

Preferably, R₂ is selected from the group consisting of hydrogen, deuterium, halogen, cyano, and hydroxy.

R₃ is selected from the group consisting of hydrogen, deuterium, halogen, cyano, hydroxy, nitro, carboxy, amino, amido, sulfonyl, C₁-C₄ alkyl, C₁-C₄ haloalkyl, C₃-C₆ cycloalkyl, C₁-C₄ alkoxy, C₁-C₄ haloalkoxy, C₃-C₆ cycloalkoxy, -C(=O)OC₁-C₄ alkyl, and -C(=O)ONH₂.

Preferably, R₃ is selected from the group consisting of hydrogen, deuterium, halogen, cyano, hydroxy, nitro, carboxyl, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, cyclohexyloxy, fluoromethyl, difluoromethyl, trifluoromethyl, dichloromethyl, trichloromethyl, fluoroethyl, difluoroethyl, trifluoroethyl, tetrafluoroethyl, pentafluoroethyl, dichloroethyl, trichloroethyl, tetrachloroethyl, pentachloroethyl, difluoropropyl, trifluoropropyl, tetrafluoropropyl, pentafluoropropyl, hexafluoropropyl, perfluoropropyl, chloropropyl, dichloropropyl, trichloropropyl, tetrachloropropyl, pentachloropropyl, hexachloropropyl, perchloropropyl, fluoromethoxy, difluoromethoxy, trifluoromethoxy, dichloromethoxy, trichloromethoxy, fluoroethoxy, difluoroethoxy, trifluoroethoxy, tetrafluoroethoxy, pentafluoroethoxy, dichloroethoxy, trichloroethoxy, tetrachloroethoxy, pentachloroethoxy, difluoropropoxy, trifluoropropoxy, tetrafluoropropoxy, pentafluoropropoxy, hexafluoropropoxy, perfluoropropoxy, chloropropoxy, dichloropropoxy, trichloropropoxy, tetrachloropropoxy, pentachloropropoxy, hexachloropropoxy, perchloropropoxy, -C(=O)O-methyl, -C(=O)O-ethyl, -C(=O)O-n-propyl, -C(=O)O-isopropyl, -C(=O)O-n-butyl, -C(=O)O-sec-butyl, -C(=O)O-tert-butyl, and -C(=O)ONH₂.

Preferably, R₃ is selected from the group consisting of hydrogen, deuterium, halogen, cyano, hydroxy, nitro, carboxyl, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, trifluoromethyl, -C(=O)O-methyl, -C(=O)O-ethyl, -C(=O)O-n-propyl, -C(=O)O-isopropyl, -C(=O)O-n-butyl, -C(=O)O-sec-butyl, -C(=O)O-tert-butyl, and -C(=O)ONH₂.

Preferably, R₄ and R₅ are each independently selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy,

Preferably, R₄ and R₅ are each independently selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexoxy,

Preferably, R₄ and R₅ are each independently selected from the group consisting of hydrogen, deuterium, halogen, cyano, and hydroxy,

Preferably, n1 is an integer selected from 0, 1, or 2.

Preferably, n2 is an integer selected from 0, 1, 2, or 3.

Preferably, n3 is an integer selected from 0, 1, 2, or 3.

Preferably, the compound of Formula I, or an isotopically labeled compound thereof, or an optical isomer, geometric isomer, tautomer or isomermixture thereof, or a pharmaceutically acceptable salt thereof, or a prodrug thereof, or a metabolite thereof are represented by the following Formula II: 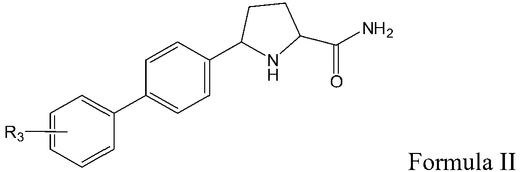 wherein, R₃ is defined the same as that in Formula I.

The compound of Formula I or II, or an isotopically labeled compound thereof, or an optical isomer, geometric isomer, tautomer or isomer mixture thereof, or a pharmaceutically acceptable salt thereof, or a prodrug thereof, or a metabolite thereof are selected from the group consisting of the following compounds:

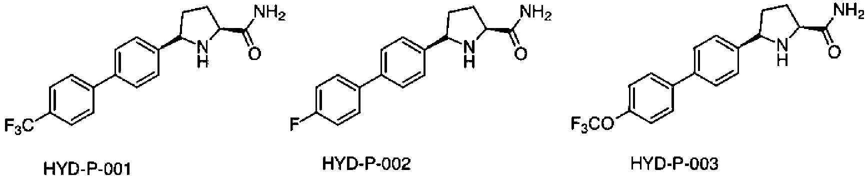

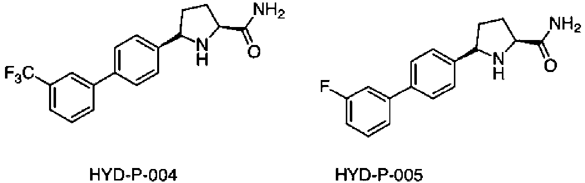

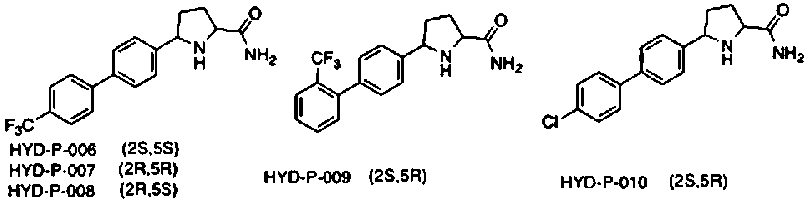

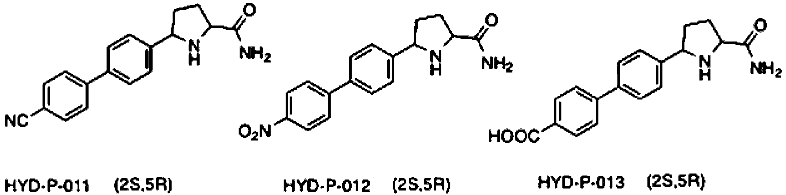

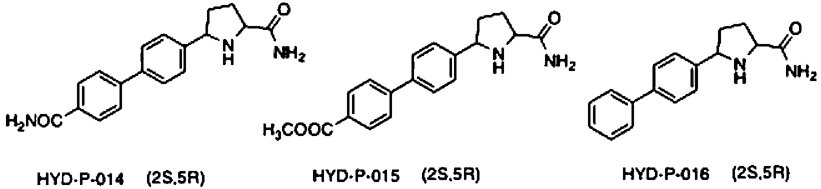

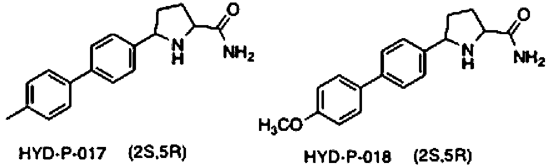

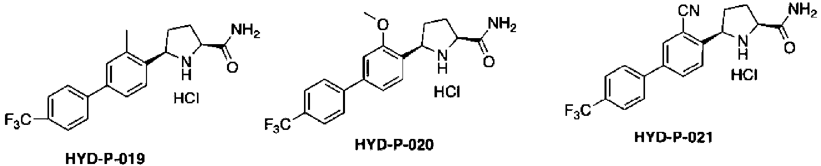

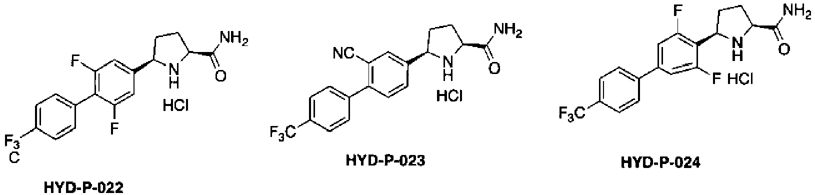

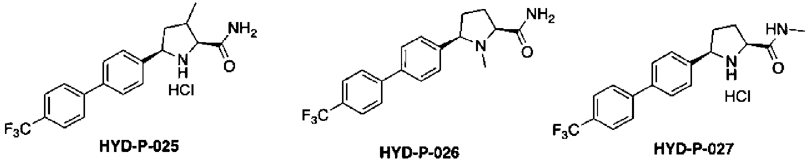

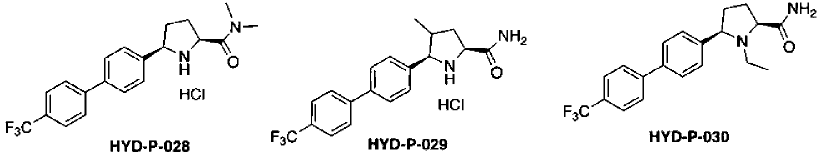

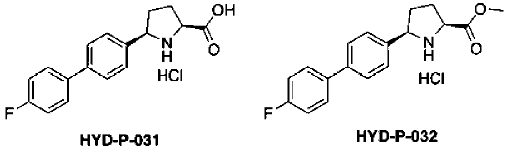

According to another aspect of the present invention, another object of the present invention is to provide a preparation method for the compound of the Formula I or II, or an isotopically labeled compound thereof, or an optical isomer, geometric isomer, tautomer or isomer mixture thereof, or a pharmaceutically acceptable salt thereof, or a prodrug thereof, or a metabolite thereof, the preparation method is carried out as follows: 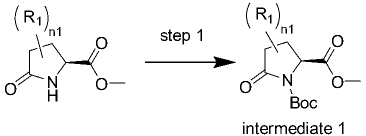 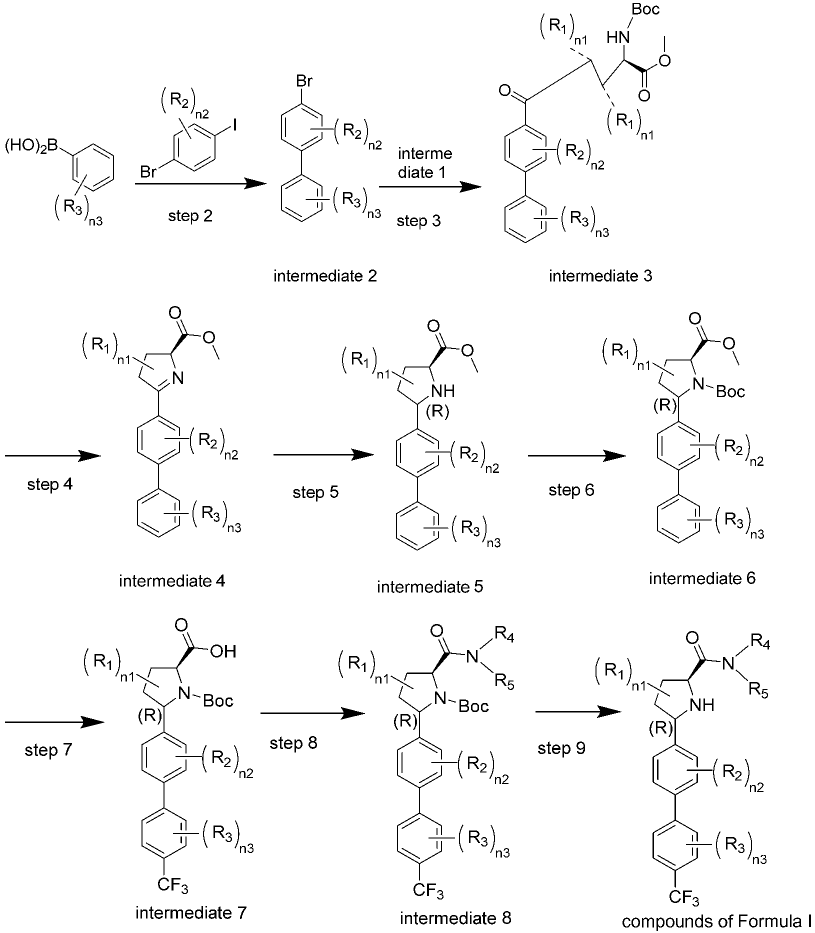
1) a methyl pyroglutamate containing a substituent R₁ is added to an organic solvent (dichloromethane, chloroform, tetrahydrofuran, etc.), and at room temperature, di-tert-butyl decarbonate (Boc₂O) and N,N-dimethylaminopyridine (DMAP) are added, to generate tert-butoxycarbonylated methyl L-pyroglutamate containing the substituent R₁, as **intermediate** 1;
2) a phenylboronic acid containing a substituent R₃ and a p-bromoiodobenzene containing a substituent R₂ with different groups are added at 25°C with sodium carbonate (Na₂CO₃) and bis(triphenylphosphine)palladium (II) chloride [Pd(PPh₃)₂Cl₂], and stirred at 90°C for 2 hours to obtain a brominated biphenyl compound as **intermediate 2;**
3) under nitrogen atmosphere, **intermediate** 2 dissolved in tetrahydrofuran is added dropwise with n-butyllithium at -78°C, and stirred for 1-2 hours while maintaining the temperature, a solution of **intermediate** 1 in dry tetrahydrofuran is added dropwise at -78°C, and the mixture is reacted for 40 minutes while maintaining the temperature, then raised to -40°C, and reacted for 2 to 3 hours to obtain **intermediate 3;**
4) **intermediate 3** is dissolved in dichloromethane at room temperature, added with trifluoroacetic acid, and reacted overnight to obtain **intermediate 4;**
5) **intermediate** 4 is dissolved in methanol under nitrogen atmosphere at room temperature, added with platinum (IV) oxide, and reacted overnight to obtain **intermediate 5;**
6) **intermediate 5** is dissolved in dichloromethane at room temperature, and added with di-tert-butyl dicarbonate to obtain **intermediate 6** whose secondary amine is protected;
7) **intermediate 6** is dissolved in tetrahydrofuran and water at room temperature, added with lithium hydroxide, raised to 50°C, and stirred for 3 to 4 hours to obtain a carboxylic acid compound as **intermediate 7;**
8) **intermediate 7** is dissolved in N,N-dimethylformamide at room temperature, added with an amine reagent containing R₄ and R₃ groups, 2-(7-azabenzotriazol-1-yl)-N,N,N',N'-tetramethyluronium hexafluorophosphate and N,N-diisopropylethylamine, and reacted at room temperature for 3 to 5 hours to obtain an amide compound as **intermediate 8;**
9) **intermediate 8** is dissolved in dichloromethane at room temperature, added with a solution of hydrogen chloride in ethyl acetate, stirred for 2-4 hours, and concentrated, and the residue is added with water, adjusted to pH 7-8 with a 5% aqueous sodium bicarbonate solution to obtain the compound of Formula 1,
wherein, the dashed line in **intermediate 3** indicates that the substituent R₁ may not be present or absent.

According to another aspect of the present invention, another object of the present invention is to provide a pharmaceutical composition comprising a therapeutically effective amount of the compound of Formula I or II of the present invention, or an isotopically labeled compound thereof, or an optical isomer, geometric isomer, tautomer or isomer mixture thereof, or a pharmaceutically acceptable salt thereof, or a prodrug thereof, or a metabolite thereof, and a pharmaceutically acceptable carrier.

According to another aspect of the present invention, another object of the present invention is to provide a use of the compound of Formula I or II of the present invention, or an isotopically labeled compound thereof, or an optical isomer, geometric isomer, tautomer or isomer mixture thereof, or a pharmaceutically acceptable salt thereof, or a prodrug thereof, or a metabolite thereof, in the preparation of a medicament for a monoamine oxidase B inhibitor, the medicament can be used for treating or preventing a disease or condition associated with monoamine oxidase B in a subject in need thereof.

Preferably, the disease or condition associated with monoamine oxidase B is a neuropsychiatric disease or condition.

More preferably, the neuropsychiatric disease or condition is selected from the group consisting of Depression, Alzheimer's Disease, Parkinson's Disease, Senile Dementia, and Narcolepsy.

According to another aspect of the present invention, another object of the present invention is to provide a kit comprising the compound of Formula I or II or an isotopically labeled compound thereof, or an optical isomer, geometric isomer, tautomer or isomer mixture thereof, or a pharmaceutically acceptable salt thereof, or a prodrug thereof, or a metabolite thereof, or the pharmaceutical composition of the present invention, and a container and an instruction for use.

According to another aspect of the present invention, another object of the present invention is to provide a method for treating a disease or condition associated with monoamine oxidase B, comprising administering to a subject in need thereof an effective amount of the compound of Formula I or II of the present invention, or an isotopically labeled compound thereof, or an optical isomer, geometric isomer, tautomer or isomer mixture thereof, or a pharmaceutically acceptable salt thereof, or a prodrug thereof, or a metabolite thereof, or the pharmaceutical composition of the present invention.

### Beneficial Effects

The present invention relates to a monoamine oxidase B inhibitor with a novel structure which is not previously reported. This series of compounds exhibits high selectivity between monoamine oxidase B and monoamine oxidase A, and can be developed as a drug to effectively reduce side effects caused by acting on monoamine oxidase A.

### Specific Embodiments

Hereinafter, the present invention will be described in detail. Before the description, it should be understood that the terms used in the present specification and the appended claims should not be construed as being limited to general and dictionary meanings, but should be interpreted based on the principle of allowing the inventor to appropriately define terms for the best explanation, according to the meanings and concepts corresponding to the technical aspects of the present invention. Therefore, the description presented herein is merely a preferred example for illustrative purposes and is not intended to limit the scope of the present invention, so it should be understood that other equivalents or modifications may be obtained therefrom without departing from the spirit and scope of the present invention.

The following examples are merely listed as examples of embodiments of the present invention and do not constitute any limitation to the present invention. Those skilled in the art can understand that modifications within the scope not departing from the spirit and concept of the present invention all fall within the protection scope of the present invention. Unless otherwise specified, the reagents and instruments used in the following examples are all commercially available products.

As used herein, the terms " comprising", "including", "having", "containing" or any other similar terms are open-ended transitional phrases which intend to cover non-exclusive contents. For example, a composition or product containing elements in the plural is not limited to those elements listed herein, but may also include other elements not expressly listed but normally inherent in the composition or product. In addition, unless expressly stated to the contrary, the term "or" is meant to be inclusive rather than exclusive. For example, the condition "A or B" is satisfied in any of the following cases: A is true (or present) and B is false (or absent), A is false (or absent) and B is true (or present), or both A and B are true (or present). In addition, herein, the terms " comprising", "including", "having", "containing" should be deemed to be understood as being specifically disclosed and to cover both the closed or semi-closed conjunctions "consisting of" and "substantially consisting of".

As used herein, all features or conditions defined in the form of numerical ranges or percentage ranges are for the sake of brevity and convenience only. Accordingly, descriptions of numerical or percentage ranges are to be regarded as covering and specifically disclosing all possible sub-ranges and individual values within the ranges, especially integer values. For example, the description of the range "1 to 8" shall be deemed to specifically disclose all sub-ranges such as 1 to 7, 2 to 8, 2 to 6, 3 to 6, 4 to 8, 3 to 8, etc., and in particular all sub-ranges defined by all integer values, and shall be deemed to specifically disclose individual values such as 1, 2, 3, 4, 5, 6, 7, 8, etc., within the range. Unless otherwise indicated, the foregoing method of interpretation applies to the entirety of the present invention, irrespective of its broad scope. When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

If quantity or other value or parameter is expressed as a range, a better range, or a series of upper and lower limits, it should be understood that all ranges limiting by any pair of upper or better values of the range and lower or better values of the range are specifically disclosed herein, whether or not such ranges are separately disclosed. In addition, when reference is made herein to a range of values, the range shall include its endpoints and all integers and fractions within the range, unless otherwise indicated.

As used herein, a numerical value is to be understood as having a precision of the effective number of digits of the value, provided that the purpose of the invention can be achieved. For example, the number 40.0 should be understood as covering a range from 39.50 to 40.49.

Where Markush group or alternative terminology is used herein to describe features or instances of the present invention, it should be understood by those skilled in the art that subgroups or any individual element of all elements of the Markush group or options may also be used to describe the present invention. For example, if X is described as "selected from the group consisting of X1, X2, and X3", it should be understood that X is X1 and X is X1 and/or X2 have been fully described and claimed. Further, in cases where Markush group or alternative terms are used to describe features or embodiments of the present invention, it should be understood by those skilled in the art that subgroups of all the elements or any combination of individual elements within a Markush group or options may also be used to describe the present invention. Accordingly, for example, if X is described as "selected from the group consisting of X1, X2, and X3" and Y is described as "selected from the group consisting of Y1, Y2, and Y3", it means that X is X1 or X2 or X3 and Y is Y1 or Y2 or Y3 is fully described and claimed.

### Definitions

Definitions of specific functional groups and chemical terms are described in more detail below. The chemical elements are identified in accordance with the Periodic Table of the Elements on the inner cover of CAS version, Handbook of Chemistry and Physics, 75th Ed., and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. The present disclosure is not intended to be limited in any manner by the exemplary listing of substituents described herein.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from the mixtures by methods known to those skilled in the art, including chiral high pressure liquid chromatography (HPLC) and the formation and crystallization of chiral salts; or preferably, isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions, p. 268 (E.L. Eliel, Ed., Univ. of Notre Dame Press, Notre Dame de Paris, IN 1972). The present disclosure additionally encompasses that the compounds described herein are individual isomers substantially free of other isomers, or are mixtures of various isomers.

The term "alkyl" refers to a straight or branched saturated hydrocarbonyl having 1 to 6 carbon atoms ("C₁-C₆ alkyl"). In some embodiments, the alkyl has 1 to 6 carbon atoms ("C₁₋₆ alkyl"). In some embodiments, the alkyl has 1 to 5 carbon atoms ("C₁₋₅ alkyl"). In some embodiments, the alkyl has 1 to 4 carbon atoms ("C₁₋₄ alkyl"). In some embodiments, the alkyl has 1 to 3 carbon atoms ("C₁₋₃ alkyl"). In some embodiments, the alkyl has 1 to 2 carbon atoms ("C₁₋₂ alkyl"). In some embodiments, the alkyl has 1 carbon atom ("C₁ alkyl"). In some embodiments, the alkyl has 2 to 6 carbon atoms ("C₂₋₆ alkyl"). Examples of C₁₋₆ alkyl include methyl (C₁), ethyl (C₂), propyl (C₃) (e.g., n-propyl, isopropyl), butyl (C₄) (e.g., n-butyl, tert-butyl, sec-butyl, iso-butyl), pentyl (C₅) (e.g., n-pentyl, 3-pentyl, neopentyl, 3-methyl-2-butyl, tert-pentyl) and hexyl (C₆) (e.g., n-hexyl). Unless otherwise indicated, each example of alkyl is independently unsubstituted ("unsubstituted alkyl") or substituted with one or more substituents (e.g., halogen, such as F) ("substituted alkyl"). In some embodiments, the alkyl is an unsubstituted C₁₋₆ alkyl, such as -CH₃. In some embodiments, the alkyl is a substituted C₁₋₆ alkyl, such as -CF₃.

"Cycloalkyl" or "carbocyclic" refers to a non-aromatic cyclic hydrocarbon group having 3 to 6 ring carbon atoms ("C3-6 carbocyclyl") and zero heteroatoms in the non-aromatic ring system. In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₃), cyclopentenyl (C₅), cyclohexyl (C₆), cyclohexenyl (C₆), cyclohexadienyl (C₆), and the like. As illustrated by the foregoing examples, in certain embodiments, the cycloalkyl group is either monocyclic ("monocyclic cycloalkyl") or contain a fused, bridged or spiro ring system such as a bicyclic system ("bicyclic cycloalkyl") and can be saturated or can be partially unsaturated. "Cycloalkyl" also includes ring systems wherein the carbocyclic ring, as defined above, is fused with one or more aryl or heteroaryl groups wherein the point of attachment is on the carbocyclic ring, and in such instances, the number of carbon atoms continue to designate the number of carbon atoms in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is a unsubstituted C₃₋₆ carbocyclyl. In certain embodiments, the carbocyclyl group is a substituted C₃₋₆ carbocyclyl.

In some embodiments, "cycloalkyl" is a monocyclic, saturated carbocyclyl group having from 3 to 6 ring carbon atoms ("C₃₋₆ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₃₋₆ cycloalkyl"). Examples of C₃₋₆ cycloalkyl groups include cyclopentyl (C₅) and cyclohexyl (C₆). Examples of C₃₋₆ cycloalkyl groups include the aforementioned C₃₋₆ cycloalkyl groups as well as cyclopropyl (C₃) and cyclobutyl (C₄). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is a unsubstituted C₃₋₆ cycloalkyl. In certain embodiments, the cycloalkyl group is a substituted C₃₋₆ cycloalkyl.

A group is optionally substituted unless expressly provided otherwise. The term "optionally substituted" refers to being substituted or unsubstituted. In certain embodiments, alkyl, alkoxy, cycloalkyl and cycloalkoxy are optionally substituted (*e.g.,* "substituted" or "unsubstituted" alkyl, "substituted" or "unsubstituted" alkoxy, "substituted" or "unsubstituted" cycloalkyl, "substituted" or "unsubstituted" cycloalkoxy). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present disclosure contemplates any and all such combinations in order to arrive at a stable compound. For purposes of the present disclosure, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

"Halo" or "halogen" refers to fluorine (fluoro, -F), chlorine (chloro, -Cl), bromine (bromo, -Br), or iodine (iodo, -I).

The term "pharmaceutically acceptable salt" refers to those salts which are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well known in the art. For example, Berge et al., describe pharmaceutically acceptable salts in detail in J. Pharmaceutical Sciences, 1977, 66, 1-19, incorporated herein by reference. Pharmaceutically acceptable salts of the compounds described herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄⁻ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "pharmaceutically acceptable carrier" refers to any formulation or carrier medium capable of delivering an effective amount of the active substance of the present invention, without interfering with the biological activity of the active substance and being non-toxic and free of side effects to the host or patient. Representative carriers include water, oils, vegetable and mineral oils, cream matrix, lotion matrix, ointment matrix, etc. These matrixes include suspending agents, viscosity-increasing agents, transdermal penetration enhancers, etc. Their formulations are well known to those skilled in the art of cosmetics or topical pharmaceuticals. For further information on carriers, it can be found from Remington: The Science and Practice of Pharmacy, 21st Ed., Lippincott, Williams & Wilkins (2005), the content of which is incorporated herein by reference.

The term "solvate" refers to forms of the compound that are associated with a solvent, usually by a solvolysis reaction. This physical association may include hydrogen bonding. Conventional solvents include water, methanol, ethanol, acetic acid, DMSO, THF, diethyl ether, and the like. The compounds described herein may be prepared, e.g., in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolatable solvates. Representative solvates include hydrates, ethanolates, and methanolates.

The term "hydrate" refers to a compound that is associated with water. Typically, the number of the water molecules contained in a hydrate of a compound is in a definite ratio to the number of the compound molecules in the hydrate. Therefore, a hydrate of a compound may be represented, for example, by the general formula R·x H₂O, wherein R is the compound, and x is a number greater than 0. A given compound may form more than one type of hydrate, including, *e.g.,* monohydrates (x is 1), lower hydrates (x is a number greater than 0 and smaller than 1, *e.g.,* hemihydrates (R·0.5 H₂O)), and polyhydrates (x is a number greater than 1, *e.g.,* dihydrates (R·2 H₂O) and hexahydrates (R·6 H₂O)).

The term "tautomer" or "tautomeric" refers to two or more interconvertible compounds resulting from at least one formal migration of a hydrogen atom and at least one change in valency (*e.g.,* a single bond to a double bond, a triple bond to a single bond, or *vice versa*)*.* The exact ratio of the tautomers depends on several factors, including temperature, solvent, and pH. Tautomerizations (*i.e.,* the reaction providing a tautomeric pair) may catalyzed by acid or base. Exemplary tautomerizations include keto-to-enol, amide-to-imide, lactam-to-lactim, enamine-to-imine, and enamine-to-(a different enamine) tautomerizations.

It is also to be understood that compounds that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

The term "prodrug" refers to compounds that have cleavable groups and become by solvolysis or under physiological conditions the compounds described herein, which are pharmaceutically active *in vivo.* Such examples include, but are not limited to, choline ester derivatives and the like, N-alkylmorpholine esters and the like. Other derivatives of the compounds described herein have activity in both their acid and acid derivative forms, but in the acid sensitive form often offer advantages of solubility, tissue compatibility, or delayed release in the mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides, and anhydrides derived from acidic groups pendant on the compounds described herein are particular prodrugs. In some cases it is desirable to prepare double ester type prodrugs such as (acyloxy)alkyl esters or ((alkoxycarbonyl)oxy)alkylesters. C₁-C₈ alkyl, C₂-C₈ alkenyl, C₂-C₈ alkynyl, aryl, C₇-C₁₂ substituted aryl, and C₇-C₁₂ arylalkyl esters of the compounds described herein may be preferred.

The term "aberrant activity" refers to activity deviating from normal activity. The term "increased activity" refers to activity higher than normal activity.

The terms "composition" and "formulation" are used interchangeably.

A "subject" to which administration is contemplated refers to a human (*i.e.,* male or female of any age group, *e.g.,* pediatric subject (*e.g.,* infant, child, or adolescent) or adult subject (*e.g.,* young adult, middle-aged adult, or senior adult)). A "patient" refers to a human subject in need of treatment of a disease.

The term "biological sample" refers to any sample including tissue samples (such as tissue sections and needle biopsies of a tissue); cell samples (*e.g.,* cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucous, tears, sweat, pus, biopsied tissue (*e.g.,* obtained by a surgical biopsy or needle biopsy), nipple aspirates, milk, vaginal fluid, saliva, swabs (such as buccal swabs), or any material containing biomolecules that is derived from a first biological sample.

The terms "administer", "administering", or "administration" refers to implanting, absorbing, ingesting, injecting, inhaling, or otherwise introducing a compound described herein, or a composition thereof, in or on a subject.

The terms "treatment", "treat", or "treating" refers to reversing, alleviating, delaying the onset of, or inhibiting the progress of a disease described herein. In some embodiments, treatment may be applied after one or more signs or symptoms of the disease have developed or have been observed. In other embodiments, treatment may be administered in the absence of signs or symptoms of the disease. For example, treatment may be administered to a susceptible subject prior to the onset of symptoms (*e.g.,* in light of a history of symptoms and/or in light of exposure to a pathogen) to delay or prevent disease occurrence. Treatment may also be continued after symptoms have resolved, for example, to delay or prevent recurrence.

An "therapeutically effective amount" of a compound described herein refers to an amount sufficient to elicit the desired biological response, *i.e.,* treating the condition. As will be appreciated by those of ordinary skill in this art, the effective amount of a compound described herein may vary depending on such factors as the desired biological endpoint, the pharmacokinetics of the compound, the condition being treated, the mode of administration, and the age and health of the subject. In certain embodiments, an effective amount is a therapeutically effective amount. In certain embodiments, an effective amount is a prophylactic treatment. In certain embodiments, an effective amount is the amount of a compound described herein in a single dose. In certain embodiments, an effective amount is the combined amounts of a compound described herein in multiple doses.

The pharmaceutical compositions described herein may be prepared by any method known in the field of pharmacology. Typically, such preparation methods involve combining the active ingredient, such as a salt of the present invention, with a carrier or excipient and/or one or more other auxiliary ingredients, and then, if necessary and/or desired, shaping and/or packaging the product into the desired single-dose or multi-dose unit.

Pharmaceutical compositions may be prepared, packaged, and/or sold in bulk as a single unit dose and/or as a plurality of single unit doses. A "unit dose" is a discrete amount of the pharmaceutical composition containing a predetermined amount of the active ingredient. The amount of the active ingredient is typically equal to the dose of the active ingredient to be administered to a subject and/or a convenient fraction thereof, such as one-half or one-third of that dose.

The relative amounts of the active ingredient, pharmaceutically acceptable excipient, and/or any other ingredients in the pharmaceutical compositions described herein may vary depending on the identity, size, and/or condition of the subject being treated, and further depending on the route by which the composition will be administered. The composition may comprise from 0.1% to 100% (w/w) of the active ingredient.

Pharmaceutically acceptable excipients suitable for use in manufacturing the pharmaceutical compositions herein include, for example, inert diluents, dispersing and/or granulating ingredients, surfactants and/or emulsifiers, disintegrants, binders, preservatives, buffers, lubricants, and/or oils. Excipients such as cocoa butter and suppository waxes, coloring ingredients, coating ingredients, sweetening ingredients, flavoring ingredients, and perfuming ingredients may also be present in the compositions.

The pharmaceutical compositions may be formulated for administration via any route, such as oral administration. Typically, the pharmaceutical compositions are solid dosage forms. However, in some embodiments, other dosage forms, such as liquid, suspension, or semi-solid dosage forms, may also be used.

Solid dosage forms for oral administration include, for example, capsules, tablets, pills, powders, and granules. In such solid dosage forms, the active ingredient is combined with at least one inert, pharmaceutically acceptable excipient or carrier (e.g., sodium citrate or dicalcium phosphate) and/or (a) a filler or extender (e.g., starch, lactose, sucrose, glucose, mannitol, and silicic acid), (b) a binder (e.g., carboxymethyl cellulose, alginates, gelatin, polyvinylpyrrolidone, sucrose, and gum arabic), (c) a humectant (e.g., glycerol), (d) a disintegrating agent (e.g., agar, calcium carbonate, potato or tapioca starch, alginic acid, certain silicates, and sodium carbonate), (e) a retarder (e.g., paraffin), (f) an absorption accelerator (e.g., quaternary ammonium compounds), (g) a wetting agent (e.g., cetyl alcohol and glyceryl monostearate), (h) an absorbent (e.g., kaolin and bentonite), and (i) a lubricant (e.g., talc, calcium stearate, magnesium stearate, solid polyethylene glycol, sodium lauryl sulfate) and a mixture thereof. For capsules, tablets, and pills, the dosage form may contain a buffering agent.

Similar types of solid compositions can also be employed as fillers in soft and hard-filled gelatin capsules using such excipients as lactose and high molecular weight polyethylene glycols. The solid dosage forms of tablets, dragees, capsules, pills, and granules can be prepared with coatings and shells, such as enteric coatings and other coatings well known in the pharmaceutical arts. They may optionally contain an opacifying agent and can also be of a composition that they release the active ingredient(s) only, or preferentially, in a certain part of the intestinal tract, optionally, in a delayed manner. Examples of embedding compositions that can be used include polymeric substances and waxes. Similar types of solid compositions can also be employed as fillers in soft and hard-filled gelatin capsules using such excipients as lactose and high molecular weight Polyethylene glycols.

The active ingredient (e.g., a compound of the present invention) may be in microencapsulated form with one or more excipients as described above. Solid dosage forms such as tablets, dragees, capsules, pills, and granules can be prepared with coatings and shells well known in the art of pharmaceutical formulation, such as enteric coatings, controlled-release coatings, and other coatings. In such solid dosage forms, the active ingredient may be mixed with at least one inert diluent (e.g., sucrose, lactose, or starch). Such dosage forms may, as per conventional practice, contain other substances besides inert diluents, for example, a tableting lubricant and other tableting aids, such as magnesium stearate and microcrystalline cellulose. In the case of capsules, tablets, and pills, the dosage forms may contain a buffering agent. They may optionally contain an opacifying agent and may have a composition that releases the active ingredient only or preferably in certain parts of the intestine, optionally in a delayed manner. Examples of encapsulating agents that can be used include polymeric substances and waxes.

Although the description of pharmaceutical compositions provided herein is primarily directed to compositions suitable for administration to humans, such compositions are generally suitable for administration to various animals. To make the composition suitable for administration to various animals, modifications to pharmaceutical compositions intended for human use are well known, and a veterinarian skilled in the art can design and/or carry out such modifications through routine experiments. For veterinary use, the compounds of the present disclosure may be administered as suitably acceptable formulations according to normal veterinary practice. A veterinarian can readily determine the most suitable dosing regimen and route of administration for a particular animal.

The compounds of the present disclosure are generally formulated in dosage unit form for ease of administration and uniformity of dosage. However, it should be understood that the total daily dosage of the compositions described herein will be determined by the physician within the scope of sound medical judgment. For any particular subject or organism, the specific therapeutically effective dose level will depend on a variety of factors, including the disease being treated and the severity of the disease; the activity of the specific active ingredient used; the specific composition used; the age, body weight, general health, gender, and diet of the subject; the time of administration, route of administration, and excretion rate of the specific active ingredient used; the duration of treatment; drugs used in combination or concurrently with the specific active ingredient used; and factors well known in the medical arts.

The present invention also includes isotopically labeled compounds of the present invention, which are identical to those compounds described herein except that one or more atoms are replaced by an atom having an atomic mass or mass number different from the atomic mass or mass number commonly found in nature. Examples of isotopes that can be incorporated into the compounds of the present invention include isotopes of hydrogen, carbon, nitrogen, oxygen, fluorine, and chlorine, such as ²H, ³H, ¹³C, ¹⁴C , ¹⁵N, ¹⁸O, ¹⁷O, ¹⁸F, and ³⁶Cl.

Certain isotopically labeled compounds of the present invention (e.g., compounds labeled with ³H and ¹⁴C) can be used in the identification of compound and/or substrate tissue distribution. Tritiated (i.e., ³H) and carbon-14 (i.e., ¹⁴C) isotopes are particularly preferred due to their ease of preparation and detection. Furthermore, substitution with heavier isotopes such as deuterium (i.e., ²H) may provide certain therapeutic benefits resulting from greater metabolic stability (e.g., increased in vivo half-life or reduced dose requirements) and may therefore be preferred in certain circumstances. Isotopically labeled compounds of the present invention can generally be prepared by following procedures analogous to those disclosed in the schemes and/or examples below, by substituting a non-isotopically labeled reagent with an isotopically labeled reagent.

The present invention also includes a kit (e.g., a drug package). The provided kit may comprise the pharmaceutical composition or salt described herein and a container (e.g., a vial, ampoule, bottle, syringe, and/or dispenser package, or other suitable container). In some embodiments, the provided kit may optionally further comprise a second container comprising a pharmaceutically acceptable carrier for diluting or suspending the pharmaceutical composition or salt of the present disclosure herein. In some embodiments, the pharmaceutical composition or salt of the present invention provided in the first container and the second container are combined to form a unit dosage form.

In some embodiments, the kit herein further includes an instruction for using the compound or pharmaceutical composition contained in the kit. The kit herein may also include information required by regulatory agencies such as the China Food and Drug Administration (SFDA). In some embodiments, the information included in the kit is prescribing information. In some embodiments, the kit and instructions provide a method for treating a disease or condition associated with monoamine oxidase B (e.g., neuropsychiatric disease or condition) and/or preventing a disease or condition associated with monoamine oxidase B (e.g., neuropsychiatric disease or condition) in a subject in need thereof. The kit described herein may include one or more additional agents described herein as a separate composition.

Furthermore, unless otherwise stated, the reagents and solvents disclosed below were purchased from Beijing InnoChem Science & Technology Co., Ltd., and ¹H NMR was performed using a Zhongke Quantum-I plus AS 400 NMR Spectrometer. LCMS was performed using a Shimadzu LCMS-IT-TOF Liquid Chromatography Mass Spectrometer. HPLC was measured using an Agilent Technologies 1200 series; purity was calculated as area % by HPLC.

### Example 1 Synthesis of (2S,5R)-5-{4-[4-(trifluoromethyl)phenyl]-phenyl}-1H-pyrrole-2-carboxamide hydrochloride

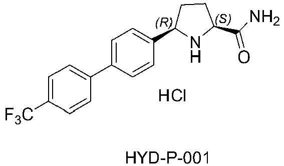

### Step 1

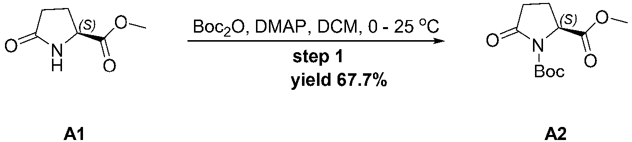

Methyl L-pyroglutamate A1 (20.0 g, 139.7 mmol) was dissolved in dichloromethane (200 mL). At room temperature, Boc₂O (45.7 g, 209.6 mmol) and DMAP (1.0 g, 8.4 mmol) were added, and the reaction mixture was stirred for 4 hours. The reaction mixture was concentrated, and the residue was purified by silica gel column chromatography (eluent: petroleum ether/ethyl acetate = 5:1) to obtain Compound A2 as a white solid (23.0 g, 67.7%).

**¹H NMR (400 MHz, CDCl₃):** *δ* ppm 4.61 (dd, *J* = 9.6, 3.2 Hz, 1H), 3.78 (s, 3H), 2.72 - 2.57 (m, 1H), 2.57 - 2.44 (m, 1H), 2.41 - 2.23 (m, 1H), 2.10 - 1.97 (m, 1H), 1.49 (s, 9H). **LCMS:** 509.2 ([2M+Na]⁺).

### Step 2

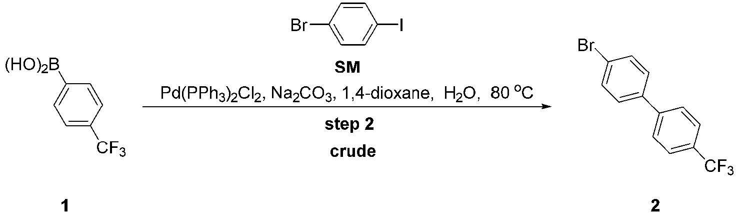

Under nitrogen atmosphere, Compound 1 (5.0 g, 26.4 mmol) was dissolved in 1,4-dioxane (75 mL) and water (25 mL). At 25 °C, SM (8.2 g, 28.9 mmol), Na₂CO₃ (8.4 g, 78.9 mmol), and Pd(PPh₃)₂Cl₂ (924 mg, 1.3 mmol) were added. The mixture was stirred at 90 °C for 2 hours. Water (100 mL) was added, and the mixture was extracted with ethyl acetate (150 mL × 2). The combined organic layers were washed respectively with water (50 mL × 2) and saturated brine (50 mL × 2), dried over anhydrous sodium sulfate, and concentrated under reduced pressure, and the crude product was purified by silica gel column chromatography (eluent: petroleum ether) to obtain Compound 2 as a white solid (8.2 g).

**¹H NMR (400 MHz, CDCl₃):** *δ* ppm 7.78 - 7.57 (m, 6H), 7.52 - 7.43 (m, 2H).

### Step 3

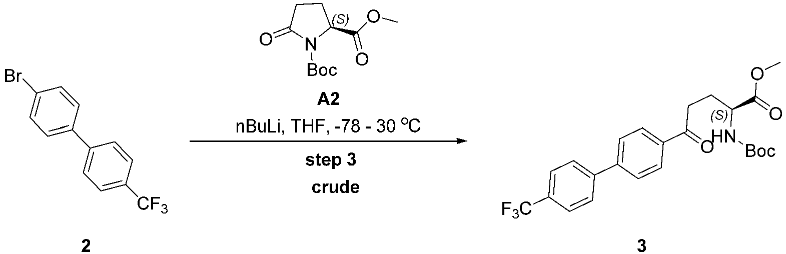

Under nitrogen atmosphere, Compound 2 (1.5 g, 5.0 mmol) was dissolved in tetrahydrofuran (8.0 mL). At -78 °C, n-Butyllithium (2.5 M in hexane, 1.8 mL, 4.5 mmol) was added dropwise. The temperature was maintained, and the mixture was stirred for 1 hour to obtain a first solution.

Compound A2 (1.0 g, 4.1 mmol, 1.0 eq) was dissolved in dry tetrahydrofuran (8.0 mL) to obtain a second solution.

The first solution was added dropwise to the second solution at -78 °C. The temperature was maintained for 40 minutes, and then the mixture was raised to -40 °C and stirred for an additional 2 hours. Saturated aqueous ammonium chloride solution (10 mL) was added. The mixture was extracted with ethyl acetate (30 mL x 3). The combined organic layers were washed with brine (15 mL), and dried over anhydrous sodium sulfate to obtain Compound 3 as a pale yellow oil (2.2 g, crude).

**LCMS: 366.1 ([M-100+H]⁺).**

### Step 4

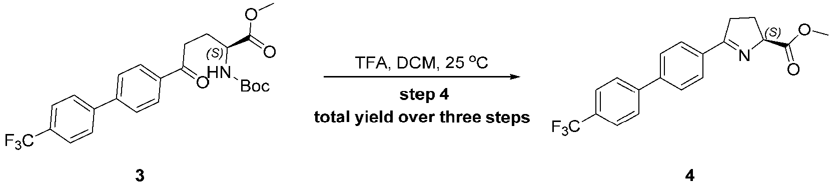

At room temperature, Compound 3 (2.2 g, 4.7 mmol) was dissolved in dichloromethane (20 mL). Trifluoroacetic acid (10.8 g, 94.5 mmol) was added, and the reaction was stirred overnight. The mixture was concentrated under reduced pressure. The residue was purified by silica gel column chromatography (eluent: petroleum ether/ethyl acetate = 5:1) to obtain Compound 4 as a white solid (1.2 g, 71.7%).

**LCMS: 348.1 ([M+H]⁺)**.

### Step 5

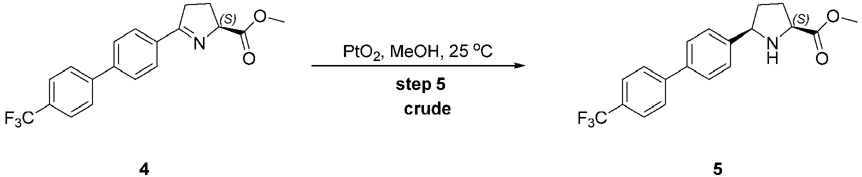

At room temperature under nitrogen atmosphere, Compound 4 (500 mg, 1.44 mmol) was dissolved in methanol (10 mL). Platinum(IV) oxide (16 mg, 0.07 mmol) was added, and the reaction was stirred overnight. The mixture was filtered, and the filtrate was concentrated to obtain Compound 5 as a white solid (500 mg, crude).

**LCMS:** 350.1 ([M+H]⁺).

### Step 6

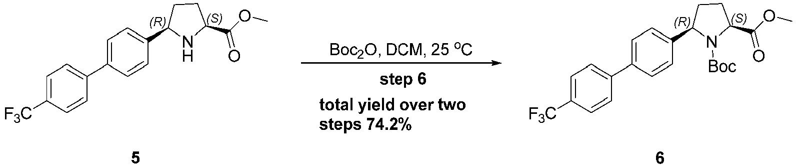

At room temperature, Compound 5 (500 mg, 1.4 mmol) was dissolved in dichloromethane (5 mL). Di-tert-butyl dicarbonate (3.1 g, 14.3 mmol) was added, and the mixture was stirred overnight. The reaction mixture was concentrated. The residue was purified by silica gel column chromatography (eluent: petroleum ether/ethyl acetate = 10:1) to obtain Compound 6 as a white solid (480 mg, 74.2%).

**LCMS:** 350.1 ([M-100+H]⁺).

### Step 7

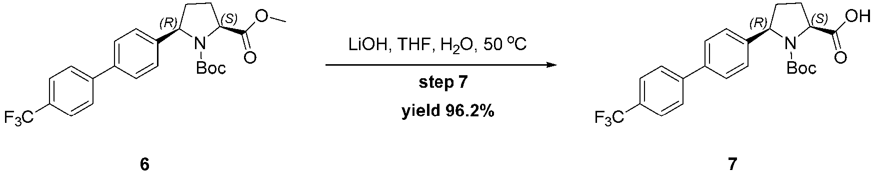

At room temperature, Compound 6 (440 mg, 0.98 mmol) was dissolved in tetrahydrofuran (7 mL) and water (3.5 mL). Lithium hydroxide (117 mg, 4.89 mmol) was added, and the mixture was heated to 50 °C and stirred for 3 hours. The mixture was concentrated under reduced pressure to remove tetrahydrofuran. Water (5 mL) was added, and the pH was adjusted to 4 with 1 N aqueous hydrochloric acid solution. The aqueous phase was extracted with methyl tert-butyl ether (10 mL x 3). The combined organic layers were washed with water (10 mL) and saturated brine (10 mL), dried over anhydrous sodium sulfate, filtered, and concentrated to obtain Compound 7 as a white solid (410 mg, 96.2%).

**LCMS:** 434.1([M-H]⁻).

### Step 8

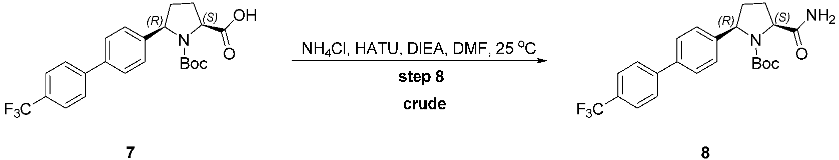

At room temperature, Compound 7 (380 mg, 0.87 mmol) was dissolved in N,N-dimethylformamide (4 mL). Ammonium chloride (117 mg, 2.6 mmol), 2-(7-azabenzotriazol-1-yl)-N,N,N',N'-tetramethyluroniumhexafluorophosphate (497 mg, 1.3 mmol), and N,N-diisopropylethylamine (563 mg, 4.4 mmol) were added. The mixture was stirred at room temperature for 3 hours. Water (10 mL) was added, and the mixture was extracted with ethyl acetate (15 mL x 2). The combined organic layers were washed with saturated brine (20 mL x 2), dried over anhydrous sodium sulfate, filtered and concentrated to obtain Compound 8 as a white oil (400 mg, crude).

**LCMS:** 457.1 ([M+Na]⁺).

### Step 9

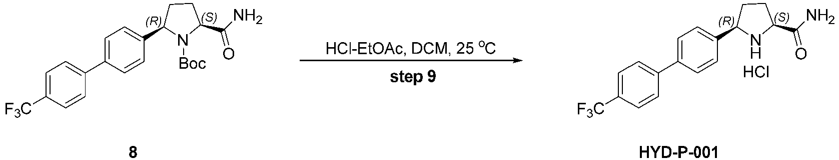

At room temperature, Compound 8 (400 mg, 0.92 mmol) was dissolved in dichloromethane (4 mL). A solution of hydrogen chloride in ethyl acetate (3.0 M, 4 mL, 12.00 mmol) was added, and the mixture was stirred for 2 hours. The mixture was concentrated. Water(5 mL) was added to the residue, and the pH was adjusted to 7-8 with a 5% aqueous sodium bicarbonate solution. The mixture was extracted with dichloromethane (5 mL x 3). The combined organic layers were dried over anhydrous sodium sulfate, filtered, and concentrated under reduced pressure. The residue was purified by preparative TLC (dichloromethane/methanol = 10:1). The obtained compound was added to a solution of hydrogen chloride in ethyl acetate (3.0 M, 5 mL) and stirred for 2 hours. The mixture was concentrated, and water (5 mL) was added. The mixture was lyophilized to obtain Compound **HYD-P-001** as a white solid (50 mg, 15.5%).

**¹H NMR (400 MHz, CDCl₃):** *δ* ppm 10.59 (brs, 1H), 8.36 (brs, 1H), 8.06 (s, 1H), 7.98 - 7.89 (m, 2H), 7.89 - 7.80 (m, 4H), 7.75 (s, 1H), 7.72 - 7.43 (m, 2H), 4.86 - 4.60 (m, 1H), 4.48 - 4.31 (m, 1H), 2.48 - 2.31 (m, 2H), 2.30 - 1.90 (m, 2H). **LCMS:** 335.1 ([M-HCl+H]⁺).

### Example 2 Synthesis of (2S,5R)-5-[4-(4-fluorophenyl)phenyl]-1H-pyrrole-2-carboxamide hydrochloride

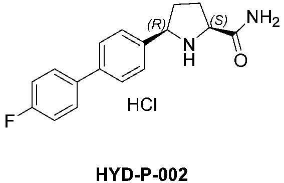

The corresponding compound was prepared in a similar manner to Example 1, except that the following corresponding reactant was used: 4-fluorophenylboronic acid (5.0 g, 35.7 mmol, 1.0 eq), to obtain (2S,5R)-5-[4-(4-fluorophenyl)phenyl]-1H-pyrrole-2-carboxamide as a white solid (40 mg, 24.0%). ¹H NMR (400 MHz, DMSO-*d₆*): *δ* ppm 10.63 (brs, 1H), 8.30 (brs, 1H), 8.06 (s, 1H), 7.85 - 7.70 (m, 5H), 7.68 - 7.57 (m, 2H), 7.32 (t, *J =* 8.8 Hz, 2H), 4.80 - 4.62 (m, 1H), 4.44 - 4.27 (m, 1H), 2.47 - 2.30 (m, 2H), 2.28 - 1.97 (m, 2H). ¹⁹F NMR (376 MHz, DMSO-*d₆*, ppm): *δ* -115.06 (1F). LCMS: 285.1 ([M-HCl+H]⁺).

### Example 3 Synthesis of (2S,5R)-5-{4-[4-(trifluoromethoxy)phenyl]phenyl}-1H-pyrrole-2-carboxamide hydrochloride

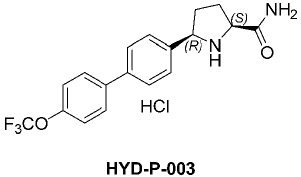

The corresponding compound was prepared in a similar manner to Example 1, except that the following corresponding reactant was used: 4-(trifluoromethoxy)phenylboronic acid (5.0 g, 24.2 mmol, 1.0 eq), to obtain (2S,5R)-5-{4-[4-(trifluoromethoxy)phenyl]phenyl}-1H-pyrrole-2-carboxamide as a white solid (74 mg, 24.6%). ¹H NMR (300 MHz, CDCl₃): *δ* ppm 10.60 (brs, 1H), 8.32 (brs, 1H), 8.06 (s, 1H), 7.90 - 7.70 (m, 5H), 7.70 - 7.58 (m, 2H), 7.48 (d, *J =* 8.4 Hz, 2H), 4.82 - 4.62 (m, 1H), 4.48 - 4.28 (m, 1H), 2.47 - 2.30 (m, 2H), 2.30 - 1.98 (m, 2H). ¹⁹F NMR (400 MHz, DMSO-*d₆*, ppm): *δ* -56.73 (3F). LCMS: 351.1 ([M-HCl+H]⁺).

### Example 4 Synthesis of (2S,5R)-5-{4-[3-(trifluoromethyl)phenyl]phenyl}-1H-pyrrole-2-carboxamide hydrochloride

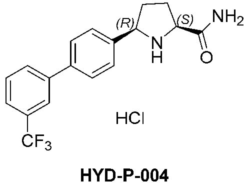

The corresponding compound was prepared in a similar manner to Example 1, except that the following corresponding reactant was used: 3-(trifluoromethyl)phenylboronic acid (5.0 g, 26.3 mmol, 1.0 eq), to obtain (2S,5R)-5-{4-[3-(trifluoromethyl)phenyl]phenyl}-1H-pyrrole-2-carboxamide as a white solid (49 mg, 11.8%). ¹H NMR (300 MHz, DMSO-*d₆*): *δ* ppm 10.66 (brs, 1H), 8.37 (brs, 1H), 8.17 - 7.93 (m, 3H), 7.90 - 7.80 (m, 2H), 7.80 - 7.55 (m, 5H), 4.85 - 4.65 (m, 1H), 4.47 - 4.30 (m, 1H), 2.47 - 2.30 (m, 2H), 2.30 - 1.93 (m, 2H). ¹⁹F NMR (300 MHz, DMSO-*d₆*, ppm): *δ* -60.98 (3F). LCMS: 335.1 ([M-HCl+H]⁺).

### Example 5 Synthesis of (2S,5R)-5-[4-(3-fluorophenyl)phenyl]-1H-pyrrole-2-carboxamide hydrochloride

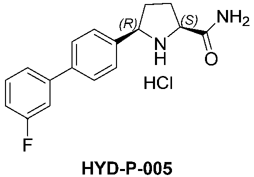

The corresponding compound was prepared in a similar manner to Example 1, except that the following corresponding reactant was used: 3-fluorophenylboronic acid (5.0 g, 35.7 mmol, 1.0 eq), to obtain (2S,5R)-5-[4-(3-fluorophenyl)phenyl]-1H-pyrrole-2-carboxamide as a white solid (55 mg, 23.2%). ¹H NMR (300 MHz, DMSO-*d₆*): *δ* ppm 10.60 (brs, 1H), 8.32 (brs, 1H), 8.05 (s, 1H), 7.90 - 7.70 (m, 3H), 7.70 - 7.60 (m, 2H), 7.60 - 7.43 (m, 3H), 7.32 - 7.13 (m, 1H), 4.83 - 4.62 (m, 1H), 4.48 - 4.26 (m, 1H), 2.47 - 2.32 (m, 2H), 2.31 - 1.90 (m, 2H). ¹⁹F NMR (282 MHz, DMSO-*d₆*, ppm): *δ* -112.69 (1F). LCMS: 285.1 ([M-HCl+H]⁺).

### Example 6 Synthesis of (2S,5S)-5-{4-[4-(trifluoromethyl)phenyl]phenyl}-1H-pyrrole-2-carboxamide hydrochloride

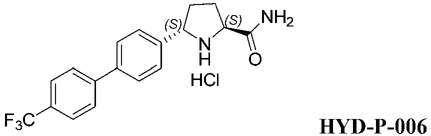

The corresponding compound was prepared in a similar manner to Example 1, except for the selection of product chirality, to obtain (2S,5S)-5-{4-[4-(trifluoromethyl)phenyl]phenyl}-1H-pyrrole-2-carboxamide as a white solid (42 mg, 70.5%). ¹H NMR (400 MHz, DMSO-*d₆*): *δ* ppm 9.36 (brs, 2H), 7.99 - 7.93 (m, 3H), 7.85 - 7.82 (m, 4H), 7.72 - 7.70 (m, 3H), 4.79 - 4.75 (m, 1H), 4.43 - 4.39 (m, 1H), 2.59 - 2.55 (m, 1H), 2.44 - 2.43 (m, 1H), 2.20 - 1.98 (m, 2H). ¹⁹F NMR (376 MHz, DMSO-*d₆*, ppm): *δ* -60.92 (3F). LCMS: 335.1 ([M-HCl+H]⁺).

### Example 7 Synthesis of (2R,5R)-5-{4-[4-(trifluoromethyl)phenyl]phenyl}-1H-pyrrole-2-carboxamide hydrochloride

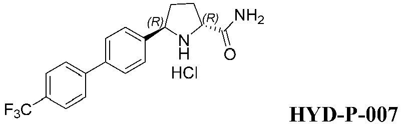

The corresponding compound was prepared in a similar manner to Example 1, except for the selection of product chirality, to obtain (2R,5R)-5-{4-[4-(trifluoromethyl)phenyl]phenyl}-1H-pyrrole-2-carboxamide as a white solid (28 mg, 32.9%). ¹H NMR (400 MHz, DMSO-*d₆*): *δ* ppm 9.36 (brs, 2H), 8.00 (brs, 1H), 7.95 - 7.93 (m, 2H), 7.85 - 7.82 (m, 4H), 7.72 - 7.68 (m, 3H), 4.79 - 4.75 (m, 1H), 4.38 - 4.34 (m, 1H), 2.58 - 2.55 (m, 1H), 2.50 - 2.43 (m, 1H), 2.31 - 1.99 (m, 2H). ¹⁹F NMR (376 MHz, DMSO-*d₆*, ppm): *δ* -60.92 (3F). LCMS: 335.1 ([M-HCl+H]⁺).

### Example 8 Synthesis of (2R,5S)-5-{4-[4-(trifluoromethyl)phenyl]phenyl}-1H-pyrrole-2-carboxamide hydrochloride

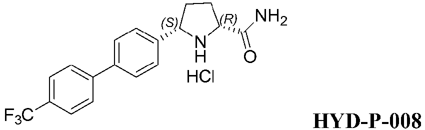

The corresponding compound was prepared in a similar manner to Example 1, except for the selection of product chirality, to obtain (2R,5S)-5-{4-[4-(trifluoromethyl)phenyl]phenyl}-1H-pyrrole-2-carboxamide as a white solid (50.1 mg, 58.6%). ¹H NMR (400 MHz, DMSO-*d₆*): *δ* ppm 10.58 (brs, 1H), 8.40 (brs, 1H), 8.06 (brs, 1H), 7.94 - 7.92 (m, 2H), 7.85 - 7.83 (m, 5H), 7.69 - 7.67 (m, 2H), 4.73 - 4.72 (m, 1H), 4.36 (m, 1H), 2.50 - 2.39 (m, 2H), 2.19 - 1.08 (m, 2H). ¹⁹F NMR (376 MHz, DMSO-*d₆*, ppm): *δ* -60.92 (3F). LCMS: 335.1 ([M-HCl+H]⁺).

### Example 9 Synthesis of (2S,5R)-5-{4-[2-(trifluoromethyl)phenyl]phenyl}-1H-pyrrole-2-carboxamide hydrochloride

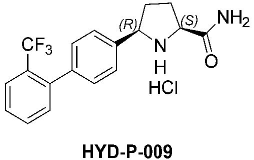

The corresponding compound was prepared in a manner similar to Example 1, except that the following corresponding reactant was used: 2-(trifluoromethyl)phenylboronic acid (5.0 g, 26.3 mmol, 1.0 eq), to obtain (2S,5R)-5-{4-[2-(trifluoromethyl)phenyl]phenyl}-1H-pyrrole-2-carboxamide as a yellow solid (23.2 mg, 38.5%). ¹H NMR (400 MHz, DMSO-*d₆*): *δ* ppm 10.97 (brs, 1H), 8.36 (brs, 1H), 8.12 (brs, 1H), 7.97 - 7.57 (m, 6H), 7.53 - 7.30 (m, 3H), 4.73 (s, 1H), 4.37 (s, 1H), 2.48 - 2.30 (m, 2H), 2.30 - 1.92 (m, 2H). LCMS: 335.1 ([M-HCl+H]⁺).

### Example 10 Synthesis of (2S,5R)-5-[4-(4-chlorophenyl)phenyl]-1H-pyrrole-2-carboxamide hydrochloride

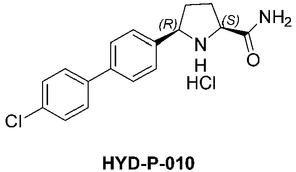

The corresponding compound was prepared in a manner similar to Example 1, except that the following corresponding reactant was used: 4-chlorophenylboronic acid (5.0 g, 32.0 mmol, 1.0 eq), to obtain (2S,5R)-5-[4-(4-chlorophenyl)phenyl]-1H-pyrrole-2-carboxamide as a white solid (42 mg, 49.9%). ¹H NMR (400 MHz, DMSO-*d₆*): *δ* ppm 10.32 (brs, 1H), 8.32 (brs, 1H), 8.02 (s, 1H), 7.88 - 7.70 (m, 5H), 7.68 - 7.60 (m, 2H), 7.60 - 7.50 (m, 2H), 4.80 - 4.64 (m, 1H), 4.40 - 4.30 (m, 1H), 2.46 - 2.30 (m, 2H), 2.28 - 2.00 (m, 2H). LCMS: 301.1 ([M-HCl+H]⁺).

### Example 11 Synthesis of (2S,5R)-5-[4-(4-cyanophenyl)phenyl]-1H-pyrrole-2-carboxamide hydrochloride

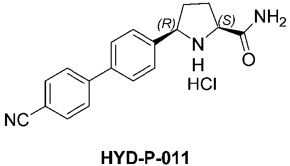

The corresponding compound was prepared in a manner similar to Example 1, except that the following corresponding reactant was used: 4-cyanophenylboronic acid (5.0 g, 34.0 mmol, 1.0 eq), to obtain (2S,5R)-5-[4-(4-cyanophenyl)phenyl]-1H-pyrrole-2-carboxamide as a white solid (35.2 mg, 41.8%). ¹H NMR (400 MHz, DMSO-*d₆*): *δ* ppm 10.69 (brs, 1H), 8.36 (brs, 1H), 8.07 (s, 1H), 8.01 - 7.90 (m, 4H), 7.85 (d, *J =* 8.4 Hz, 2H), 7.76 (s, 1H), 7.69 (d, *J =* 8.4 Hz, 2H), 4.73 (s, 1H), 4.37 (s, 1H), 2.47 - 2.30 (m, 2H), 2.27 - 2.00 (m, 2H). LCMS: 292.1 ([M-HCl+H]⁺).

### Example 12 Synthesis of (2S,5R)-5-[4-(4-nitrophenyl)phenyl]-1H-pyrrole-2-carboxamide hydrochloride

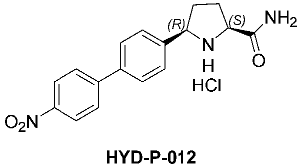

The corresponding compound was prepared in a similar manner to Example 1, except that the following corresponding reactant was used: 4-nitrophenylboronic acid (5.0 g, 29.8 mmol, 1.0 eq), to obtain (2S,5R)-5-[4-(4-nitrophenyl)phenyl]-1H-pyrrole-2-carboxamide as a white solid (25.6 mg, 59.2%). ¹H NMR (400 MHz, DMSO-*d₆*): *δ* ppm 10.85 (brs, 1H), 8.32 (d, *J =* 8.4 Hz, 2H), 8.09 (s, 1H), 8.01 (d, *J* = 8.8 Hz, 2H), 7.88 (d, *J* = 8.0 Hz, 2H), 7.80 - 7.62 (m, 3H), 4.83 - 4.67 (m, 1H), 4.44 - 4.30 (m, 1H), 2.47 - 2.30 (m, 2H), 2.28 - 1.93 (m, 2H). LCMS: 312.1 ([M-HCl+H]⁺).

### Example 13 Synthesis of (2S,5R)-5-[4-(4-carboxyphenyl)phenyl]-1H-pyrrole-2-carboxamide hydrochloride

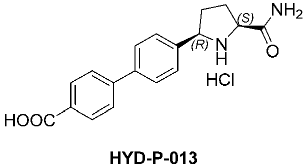

The corresponding compound was prepared in a similar manner to Example 1, except that the following corresponding reactant was used: 4-carboxyphenylboronic acid (5.0 g, 29.6 mmol, 1.0 eq), to obtain (2S,5R)-5-[4-(4-carboxyphenyl)phenyl]-1H-pyrrole-2-carboxamide as a white solid (46.3 mg, 53.8%). ¹H NMR (300 MHz, DMSO-*d₆*): *δ* ppm 13.01 (brs, 1H), 10.38 (brs, 1H), 8.32 (brs, 1H), 8.10 - 7.93 (m, 3H), 7.90 - 7.80 (m, 4H), 7.76 (s, 1H), 7.66 (d, *J* =8.1 Hz, 2H), 4.82 - 4.62 (m, 1H), 4.43 - 4.27 (m, 1H), 2.48 - 2.30 (m, 2H), 2.30 - 1.90 (m, 2H). LCMS: 311.1 ([M-HCl+H]⁺).

### Example 14 Synthesis of (2S,5R)-5-[4-(4-carbamoylphenyl)phenyl]-1H-pyrrole-2-carboxamide hydrochloride

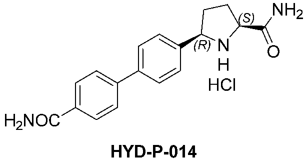

The corresponding compound was prepared in a similar manner to Example 1, except that the following corresponding reactant was used: 4-carbamoylphenylboronic acid (5.0 g, 29.6 mmol, 1.0 eq), to obtain (2S,5R)-5-[4-(4-carbamoylphenyl)phenyl]-1H-pyrrole-2-carboxamide as a white solid (23.5 mg, 54.5%). ¹H NMR (400 MHz, DMSO-*d₆*): *δ* ppm 10.45 (brs, 1H) 8.35 (brs, 1H), 8.12 - 7.92 (m, 4H), 7.90 - 7.70 (m, 5H), 7.65 (d, *J =* 8.0 Hz, 2H), 7.41 (s, 1H), 4.80 - 4.64 (m, 1H), 4.40 - 4.30 (m, 1H), 2.46 - 2.30 (m, 2H), 2.27 - 1.93 (m, 2H). LCMS: 310.1 ([M-HCl+H]⁺).

### Example 15 Synthesis of (2S,5R)-5-[4-(4-(methoxycarbonylphenyl)phenyl) phenyl]-1H-pyrrole-2-carboxamide hydrochloride

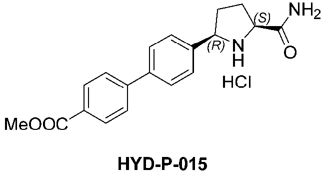

The corresponding compound was prepared in a manner similar to Example 1, except that the following corresponding reactant was used: 4-chlorophenylboronic acid (5.0 g, 27.8 mmol, 1.0 eq), to obtain (2S,5R)-5-[4-(4-methoxycarbonylphenyl)phenyl]-1H-pyrrole-2-carboxamide as a white solid (50.1 mg, 58.8%). ¹H NMR (400 MHz, DMSO-*d₆*): *δ* ppm 10.60 (brs, 1H), 8.37 (brs, 1H), 8.12 - 7.98 (m, 3H), 7.93 - 7.80 (m, 4H), 7.75 (s, 1H), 7.68 (d, *J =* 8.4 Hz, 2H), 4.80 - 4.65 (m, 1H), 4.43 - 4.30 (m, 1H), 3.88 (s, 3H), 2.47 - 2.30 (m, 2H), 2.28 - 2.20 (m, 2H). LCMS: 325.2 ([M-HCl+H]⁺).

### Example 16 Synthesis of (2S,5R)-5-[4-biphenyl]-1H-pyrrole-2-carboxamide hydrochloride

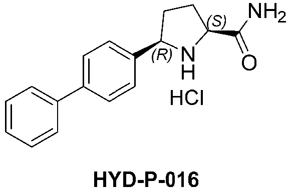

The corresponding compound was prepared in a manner similar to Example 1, except that the following corresponding reactant was used: phenylboronic acid (5.0 g, 41.0 mmol, 1.0 eq), to obtain (2S,5R)-5-[4-biphenyl]-1H-pyrrole-2-carboxamide as a white solid (25.0 mg, 50.4%). ¹H NMR (400 MHz, DMSO-*d₆*): *δ* ppm 10.48 (brs, 1H), 8.30 (brs, 1H), 8.05 (s, 1H), 7.85 - 7.60 (m, 7H), 7.49 (t, *J =* 7.2 Hz, 2H), 7.45 - 7.35 (m, 1H), 4.80 - 4.63 (m, 1H), 4.43 - 4.30 (m, 1H), 2.47 - 2.30 (m, 2H), 2.28 - 2.00 (m, 2H). LCMS: 267.1 ([M-HCl+H]⁺).

### Example 17 Synthesis of (2S,5R)-5-[4-(4-methylphenyl)phenyl]-1H-pyrrole-2-carboxamide hydrochloride

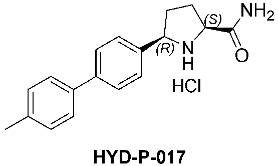

The corresponding compound was prepared in a manner similar to Example 1, except that the following corresponding reactant was used: 4-methylphenylboronic acid (5.0 g, 36.8 mmol, 1.0 eq), to obtain (2S,5R)-5-[4-(4-methylphenyl)phenyl]-1H-pyrrole-2-carboxamide as a white solid (26.2 mg, 44.6%). ¹H NMR (400 MHz, DMSO-*d₆*): *δ* ppm 10.48 (brs, 1H), 8.29 (brs, 1H), 8.04 (s, 1H), 7.85 - 7.68 (m, 3H), 7.67 - 7.50 (m, 4H), 7.29 (d, *J =* 7.6 Hz, 2H), 4.78 - 4.62 (m, 1H), 4.42 - 4.28 (m, 1H), 2.45 - 2.30 (m, 5H), 2.27 - 2.00 (m, 2H). LCMS: 281.2 ([M-HCl+H]⁺).

### Example 18 Synthesis of (2S,5R)-5-[4-(4-methoxyphenyl)phenyl]-1H-pyrrole-2-carboxamide hydrochloride

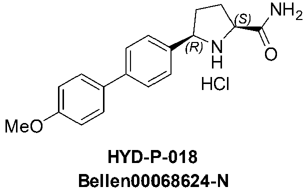

The corresponding compound was prepared in a similar manner to Example 1, except that the following corresponding reactant was used: 4-methoxyphenylboronic acid (5.0 g, 32.9 mmol, 1.0 eq), to obtain (2S,5R)-5-[4-(4-methoxyphenyl)phenyl]-1H-pyrrole-2-carboxamide as a white solid (25.4 mg, 33.1%). ¹H NMR (400 MHz, DMSO-*d₆*): *δ* ppm 10.19 (brs, 1H), 8.29 (brs, 1H), 8.00 (s, 1H), 7.76 (s, 1H), 7.70 (d, *J* = 8.4 Hz, 2H), 7.68 - 7.62 (m, 2H), 7.58 (d, *J* = 8.4 Hz, 2H), 7.10 - 7.00 (m, 2H), 4.73 - 4.62 (m, 1H), 4.38 - 4.28 (m, 1H), 3.80 (s, 3H), 2.46 - 2.30 (m, 2H), 2.25 - 2.00 (m, 2H). LCMS: 297.2 ([M-HCl+H]⁺).

### Example 19 Synthesis of (2S,5R)-5-[2-methyl-4-(4-(trifluoromethyl)phenyl)phenyl]-1H-pyrrole-2-carboxamide hydrochloride

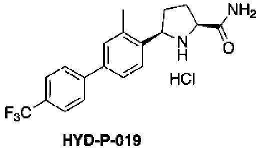

The corresponding compound was prepared in a similar manner to Example 1, except that the following corresponding reactant was used: 2-iodo-5-bromotoluene (8.6 g, 28.9 mmol, 1.1 eq), to obtain (2S,5R)-5-[2-methyl-4-(4-(trifluoromethyl)phenyl)phenyl]-1H-pyrrole-2-carboxamide hydrochloride as a white solid (29 mg, 34.3%). ¹H NMR (400 MHz, DMSO-*d₆*): *δ* ppm 10.19 (brs, 1H), 8.27 (brs, 1H), 8.01 (s, 1H), 7.77 (s, 1H), 7.71 (d, *J* = 8.4 Hz, 2H), 7.56 (d, *J =* 8.4 Hz, 2H), 7.10 - 7.00 (m, 2H), 4.70 - 4.61 (m, 1H), 4.39 - 4.21 (m, 1H), 3.10 (s, 3H), 2.39 - 2.20 (m, 2H), 2.13 - 1.96 (m, 2H). LCMS: 348.6 ([M-HCl+H]⁺).

### Example 20 Synthesis of (2S,5R)-5-[2-methoxy-4-(4-(trifluoromethyl)phenyl) phenyl]-1H-pyrrole-2-carboxamide hydrochloride

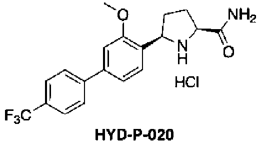

The corresponding compound was prepared in a similar manner to Example 1, except that the following corresponding reactant was used: 1-iodo-2-methoxy-4-bromobenzene (9.0 g, 28.9 mmol, 1.1 eq), to obtain (2S,5R)-5-[2-methoxy-4-(4-(trifluoromethyl)phenyl)phenyl]-1H-pyrrole-2-carboxamide hydrochloride as a white solid (30.0 mg, 39.1%). ¹H NMR (400 MHz, DMSO-*d₆*): *δ* ppm 10.17 (brs, 1H), 8.29 (brs, 1H), 8.00 (s, 1H), 7.76 (s, 1H), 7.70 (d, *J =* 8.4 Hz, 2H), 7.58 (d, *J =* 8.4 Hz, 2H), 7.10 - 7.00 (m, 2H), 4.73 - 4.62 (m, 1H), 4.38 - 4.28 (m, 1H), 3.80 (s, 3H), 2.46 - 2.30 (m, 2H), 2.25 - 2.00 (m, 2H). LCMS: 365.1 ([M-HCl+H]⁺).

### Example 21 Synthesis of (2S,5R)-5-[2-cyano-4-(4-(trifluoromethyl)phenyl) phenyl]-1H-pyrrole-2-carboxamide hydrochloride

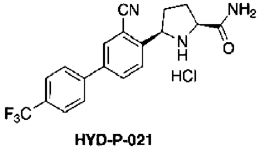

The corresponding compound was prepared in a similar manner to Example 1, except the following corresponding reactant was used: 5-bromo-2-iodobenzonitrile (8.9 g, 28.9 mmol, 1.1 eq), to obtain (2S,5R)-5-[2-cyano-4-(4-(trifluoromethyl)phenyl)phenyl]-1H-pyrrole-2-carboxamide hydrochloride as a white solid (30.1 mg, 41.9%). ¹H NMR (400 MHz, DMSO-*d₆*): *δ* ppm 10.19 (brs, 1H), 8.29 (brs, 1H), 8.00 (s, 1H), 7.78 (s, 1H), 7.72 (d, *J =* 8.4 Hz, 2H), 7.59 (d, *J =* 8.4 Hz, 2H), 7.10 - 7.02 (m, 2H), 4.73 - 4.62 (m, 1H), 4.38 - 4.28 (m, 1H), 2.46 - 2.30 (m, 2H), 2.25 - 2.00 (m, 2H). LCMS: 360.3([M-HCl+H]⁺).

### Example 22 Synthesis of (2S,5R)-5-[3,5-difluoro-4-(4-(trifluoromethyl)phenyl) phenyl]-1H-pyrrole-2-carboxamide hydrochloride

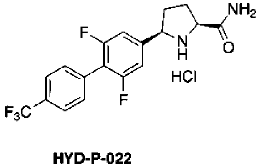

The corresponding compound was prepared in a similar manner to Example 1, except the following corresponding reactant was used: 2-bromo-1,3-difluoro-5-iodobenzene (9.2 g, 28.9 mmol, 1.1 eq), to obtain (2S,5R)-5-[3,5-difluoro-4-(4-(trifluoromethyl)phenyl)phenyl]-1H-pyrrole-2-carboxamide hydrochloride as a white solid (36.1 mg, 40.2%). ¹H NMR (400 MHz, DMSO-*d₆*): *δ* ppm 10.19 (brs, 1H), 8.29 (brs, 1H), 8.01 (s, 1H), 7.70 (d, *J* = 8.2 Hz, 2H), 7.58 (d, *J* = 8.2 Hz, 2H), 7.21(s, 2H), 4.73 - 4.62 (m, 1H), 4.38 - 4.28 (m, 1H), 2.46 - 2.35 (m, 2H), 2.22 - 2.01 (m, 2H). LCMS: 371.3 ([M-HCl+H]⁺).

### Example 23 Synthesis of (2S,5R)-5-[3-cyano-4-(4-(trifluoromethyl)phenyl) phenyl]-1H-pyrrole-2-carboxamide hydrochloride

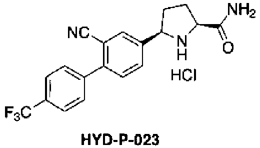

The corresponding compound was prepared in a similar manner to Example 1, except the following corresponding reactant was used: 2-bromo-5-iodobenzonitrile (8.9 g, 28.9 mmol, 1.1 eq), to obtain (2S,5R)-5-[3-cyano-4-(4-(trifluoromethyl)phenyl)phenyl]-1H-pyrrole-2-carboxamide hydrochloride as a white solid (30.3 mg, 40.2%). ¹H NMR (400 MHz, DMSO-*d₆*): *δ* ppm 10.19 (brs, 1H), 8.29 (brs, 1H), 8.00 (s, 1H), 7.76 (s, 1H), 7.70 (d, *J =* 8.4 Hz, 2H), 7.68 - 7.62 (m, 2H), 7.58 (d, *J =* 8.4 Hz, 2H), 7.10 - 7.00 (m, 2H), 4.73 - 4.62 (m, 1H), 4.38 - 4.28 (m, 1H), 3.80 (s, 3H), 2.46 - 2.30 (m, 2H), 2.25 - 2.00 (m, 2H). LCMS: 360.3 ([M-HCl+H]⁺).

### Example 24 Synthesis of (2S,5R)-5-[2,6-difluoro-4-(4-(trifluoromethyl)phenyl)phenyl]-1H-pyrrole-2-carboxamide hydrochloride

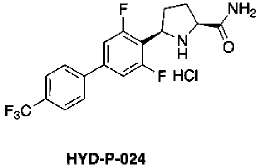

The corresponding compound was prepared in a similar manner to Example 1, except the following corresponding reactant was used: 2,6-difluoro-4-bromoiodobenzene (9.2 g, 28.9 mmol, 1.1 eq), to obtain (2S,5R)-5-[2,6-difluoro-4-(4-trifluoromethylphenyl)phenyl]-1H-pyrrole-2-carboxamide hydrochloride as a white solid (34.5 mg, 42.4%). ¹H NMR (400 MHz, DMSO-*d₆*): *δ* ppm 10.19 (brs, 1H), 8.29 (brs, 1H), 8.01 (s, 1H), 7.70 (d, *J* = 8.4 Hz, 2H), 7.58 (d, *J* = 8.4 Hz, 2H), 7.41 (s, 2H), 4.73 - 4.62 (m, 1H), 4.38 - 4.28 (m, 1H), 3.80 (s, 3H), 2.46 - 2.30 (m, 2H), 2.25 - 2.00 (m, 2H). LCMS: 371.3 ([M-HCl+H]⁺).

### Example 25 Synthesis of (2S,5R)-5-[2-methoxy-4-(4-trifluoromethylphenyl)phenyl]-3-methyl-1H-pyrrole-2-carboxa mide hydrochloride

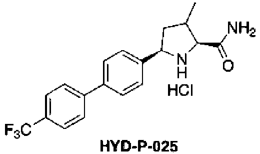

The corresponding compound was prepared in a similar manner to Example 1, except the following corresponding reactant was used: 4-methyl-L-pyroglutamic acid (21.9 g, 139.7 mmol), to obtain (2S,5R)-5-[4-(4-trifluoromethylphenyl)phenyl]-3-methyl-1H-pyrrole-2-methylcarboxamide hydrochloride as a white solid (26.5 mg, 38.6%). ¹H NMR (400 MHz, CDCl₃): *δ* ppm 10.59 (brs, 1H), 8.36 (brs, 1H), 8.06 (s, 1H), 7.98 - 7.89 (m, 2H), 7.89 - 7.80 (m, 4H), 7.75 (s, 1H), 7.72 - 7.43 (m, 2H), 4.86 - 4.60 (m, 1H), 4.48 - 4.31 (m, 1H), 2.48 - 2.31 (m, 2H), 2.30 - 1.90 (m, 1H), 0.93(s, 3H). LCMS: 349.3 ([M-HCl+H]⁺).

### Example 26 Synthesis of (2S,5R)-5-[2-methoxy-4-(4-trifluoromethylphenyl)phenyl]-N-methyl-pyrrole-2-carboxami de

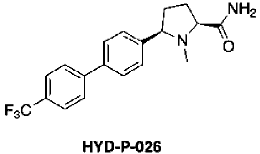

The corresponding compound was prepared in a similar manner to Example 1, except the following corresponding reactant was used: iodomethane (42.6 mg, 0.3 mmol, 1.5 eq), to obtain (2S,5R)-5-[2-methoxy-4-(4-trifluoromethylphenyl)phenyl]-N-methyl-pyrrole-2-carboxamide as a white solid (30.5 mg, 43.8%). ¹H NMR (400 MHz, DMSO-*d₆*): *δ* ppm 10.19 (brs, 1H), 8.01 (s, 1H), 7.70 (d, *J =* 8.4 Hz, 2H), 7.58 (d, *J =* 8.4 Hz, 2H), 7.41 (s, 2H), 4.73 - 4.62 (m, 1H), 4.38 - 4.28 (m, 1H), 2.76 (s, 3H), 2.46 - 2.30 (m, 2H), 2.25 - 2.00 (m, 2H). LCMS: 349.4 ([M-HCl+H]⁺).

### Example 27 Synthesis of (2S,5R)-5-[4-(4-trifluoromethylphenyl)phenyl]-1H-pyrrole-2-methylcarboxamide hydrochloride

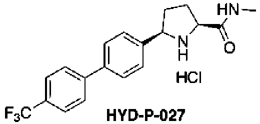

The corresponding compound was prepared in a similar manner to Example 1, except that the following corresponding reactant was used: methylamine (3.1 mL, 33%, 930.0 mg, 30.0 mmol, 1.1 eq), to obtain (2S,5R)-5-[4-(4-trifluoromethylphenyl)phenyl]-1H-pyrrole-2-methylcarboxamide hydrochloride as a white solid (30.5 mg, 43.8%).

**¹H NMR (400 MHz, CDCl₃):** *δ* ppm 10.56 (brs, 1H), 8.37 (brs, 1H), 8.03 (s, 1H), 7.98 - 7.88 (m, 2H), 7.89 - 7.80 (m, 4H), 7.76 (s, 1H), 7.72 - 7.45 (m, 2H), 4.86 - 4.61 (m, 1H), 4.48 - 4.31 (m, 1H), 2.80 (s, 3H), 2.46 - 2.31 (m, 2H), 2.310 - 1.90 (m, 2H). **LCMS:** 349.4 ([M-HCl+H]⁺).

### Example 28 Synthesis of (2S,5R)-5-[2-methoxy-4-(4-trifluoromethylphenyl)phenyl]-1H-pyrrole-2-dimethylcarboxa mide hydrochloride

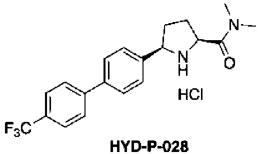

The corresponding compound was prepared in a similar manner to Example 1, except that the following corresponding reactant was used: dimethylamine (3.3 mL, 40% in methanol, 1350.0 mg, 30.0 mmol, 1.1 eq), to obtain (2S,5R)-5-[2-methoxy-4-(4-trifluoromethylphenyl)phenyl]-1H-pyrrole-2-dimethylcarboxamide hydrochloride as a white solid (28.7 mg, 37.8%).

**¹H NMR (400 MHz, CDCl₃):** *δ* ppm 10.56 (brs, 1H), 8.37 (brs, 1H), 8.06 (s, 1H), 7.98 - 7.89 (m, 2H), 7.89 - 7.80 (m, 4H), 7.76 (s, 1H), 7.72 - 7.45 (m, 2H), 4.86 - 4.61 (m, 1H), 4.48 - 4.31 (m, 1H), 2.80 (s, 6H), 2.46 - 2.31 (m, 2H), 2.310 - 1.90 (m, 2H). **LCMS:** 363.4 ([M-HCl+H]⁺).

### Example 29 Synthesis of (2S,5R)-5-[4-(4-trifluoromethylphenyl)phenyl]-4-methyl-1H-pyrrole-2-methylcarboxamid e hydrochloride

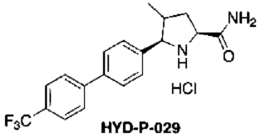

The corresponding compound was prepared in a similar manner to Example 1, except that the following corresponding reactant was used: 3-methyl-L-pyroglutamic acid (21.9 g, 139.7 mmol), to obtain (2S,5R)-5-[4-(4-trifluoromethylphenyl)phenyl]-4-methyl-1H-pyrrole-2-methylcarboxamide hydrochloride as a white solid (25.5 mg, 36.6%). **¹H NMR (400 MHz, CDCl₃):** *δ* ppm 10.59 (brs, 1H), 8.36 (brs, 1H), 8.06 (s, 1H), 7.98 - 7.89 (m, 2H), 7.89 - 7.80 (m, 4H), 7.75 (s, 1H), 7.72 - 7.43 (m, 2H), 4.86 - 4.60 (m, 1H), 4.48 - 4.31 (m, 1H), 2.48 - 2.31 (m, 1H), 2.30 - 1.90 (m, 2H), 0.93(s, 3H). **LCMS:** 349.3 ([M-HCl+H]⁺).

### Example 30 Synthesis of (2S,5R)-5-[4-(4-trifluoromethylphenyl)phenyl]-N-ethyl-pyrrole-2-methylcarboxamide

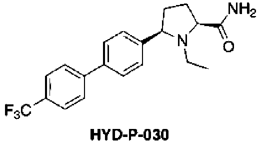

The corresponding compound was prepared in a similar manner to Example 1, except that the following corresponding reactant was used: iodoethane (46.7 mg, 0.3 mmol, 1.5 eq), to obtain (2S,5R)-5-[4-(4-trifluoromethylphenyl)phenyl]-N-ethyl-pyrrole-2-methylcarboxamide as a white solid (32.6 mg, 43.8%). ¹H NMR (400 MHz, DMSO-*d₆*): *δ* ppm 10.19 (brs, 1H), 8.01 (s, 1H), 7.70 (d, *J =* 8.4 Hz, 2H), 7.58 (d, *J =* 8.4 Hz, 2H), 7.41 (s, 2H), 4.73 - 4.62 (m, 1H), 4.38 - 4.28 (m, 1H), 2.68 (m, 2H), 2.46 - 2.30 (m, 2H), 2.25 - 2.00 (m, 2H),1.02 (m, 3H) LCMS: 363.4 ([M-HCl+H]⁺).

### Example 31 Synthesis of (2S,5R)-5-[4-(4-fluorophenyl)phenyl]-1H-pyrrole-2-carboxylic acid hydrochloride

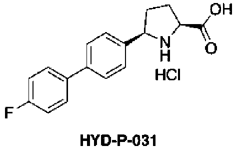

The corresponding compound was prepared in a similar manner to Example 1, except that the methyl carboxylate was not subjected to amination, but directly hydrolyzed to be a carboxylic acid, to obtain (2S,5R)-5-[4-(4-fluorophenyl)phenyl]-1H-pyrrole-2-carboxylic acid hydrochloride as a white solid (35.5 mg, 28.2%). ¹H NMR (400 MHz, DMSO-d₆): *δ* ppm 12.39 (brs, 1H), 10.63 (brs, 1H), 8.30 (brs, 1H), 7.85 - 7.70 (m, 5H), 7.68 - 7.57 (m, 2H), 7.32 (t, J = 8.8 Hz, 2H), 4.80 - 4.62 (m, 1H), 4.44 - 4.27 (m, 1H), 2.47 - 2.30 (m, 2H), 2.28 - 1.97 (m, 2H). LCMS: 286.3 ([M-HCl+H]⁺).

### Example 32 Synthesis of (2S,5R)-5-[4-(4-fluorophenyl)phenyl]-1H-pyrrole-2-carboxylic acid methyl ester hydrochloride

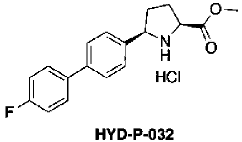

The corresponding compound was prepared in a similar manner to Example 1, except that the methyl carboxylate was reacted as the effective group and was not subjected to amination, to obtain (2S,5R)-5-[4-(4-fluorophenyl)phenyl]-1H-pyrrole-2-carboxylic acid methyl ester hydrochloride as a white solid (45.5 mg, 46.2%). **¹H NMR (400 MHz, DMSO-d₆):** *δ* **ppm** 10.68 (brs, 1H), 8.31 (brs, 1H), 7.83 - 7.70 (m, 5H), 7.64 - 7.55 (m, 2H), 7.31 (t, J = 8.4 Hz, 2H), 4.80 - 4.62 (m, 1H), 4.51 (s, 3H), 4.44 - 4.27 (m, 1H), 2.47 - 2.30 (m, 2H), 2.28 - 1.97 (m, 2H). LCMS: 300.3 ([M-HCl+H]⁺).

### Test Example 1

### 1. Human Recombinant MAO-A/B Inhibitory Activity Assay

Full-length human recombinant MAO-A and MAO-B proteins were both expressed in Baculovirus infected Sf 9 cells, containing an N-terminal FLAG tag, with a molecular weight of 59.8 kDa. The recombinant proteins were purified by SDS-PAGE, with purity > 85%, and the protein concentration was 0.3 µg/µL for both. A 40 µM substrate was incubated with the MAO protein at room temperature for 1 hour. The product generated by the oxidative deamination reaction between the MAO and the substrate was incubated with a fluorescent luminescent agent for 0.5 hours, and then the activity of the MAO was detected using the Promega MAO-Glo TM detection method.

### 2. Test Method

Human recombinant monoamine oxidase A/B expressed in Sf 9 cells was used. During the experiment, the recombinant monoamine oxidase A/B was first dissolved in a HEPES buffer (0.8% NaCl, 0.037% KCl, 0.0135% Na₂HPO₄·2H₂O, 0.1% Glucan, 0.5% HEPES, pH 7.0). 10 µL of the monoamine oxidase A/B solution was added to a 384-well plate, followed by the addition of the test compound (final DMSO concentration of 1%) in each well for 10 concentration gradients. The positive control used was Clorgyline or Selegiline hydrochloride. The plate was incubated at room temperature for 15 minutes. 10 µL of a substrate solution (40 µM, with tyramine and benzylamine as substrates for MAO-A and MAO-B, respectively) was added to each well, followed by incubation at room temperature for 60 minutes. Subsequently, 20 µL of luciferin detection reagent was added to each well, mixed thoroughly, and incubated at room temperature for 20 minutes to generate a stable fluorescent signal. The fluorescent signal was read using a fluorescence microplate reader, and the values were expressed as relative light units (RLU). The experiment was designed with duplicate wells, and the average value was used to calculate the enzyme activity inhibition rate using the following formula:$Inh % = \left(Max - Signal\right) / \left(Max - Min\right) ∗ 100$
where Max is the value detected at the maximum concentration of the sample, Min is the value detected at the minimum concentration of the sample, and Signal is the value detected at the current concentration of the sample. Based on the experimental results, the IC₅₀ was calculated using the software GraphPad Prism 5 with the following formula: $Y = Bottom + \left(Top-Bottom\right) / \left(1+{10}^{∧}\left(\left(Log IC50-X\right)*Hill Slope\right)\right)$
where X: Logarithm of compound concentration, Y: Inhibition rate (%), Top and Bottom: Plateau units are the same as Y, Hill Slope: Slope factor or curve slope.

**Table 1. Activity Data IC₅₀ (nM)**

| Compound. | MAO-A IC₅₀ (nM) | MAO-B IC₅₀ (nM) |
|---|---|---|
| 1 | >10000 | 83.01 |
| 2 | >10000 | 1505 |
| 3 | >10000 | 198.5 |
| 4 | >10000 | 476.7 |
| 5 | >10000 | 3048 |
| 6 | >10000 | 103.9 |
| 7 | >10000 | 147.1 |
| 8 | 9974 | 562.3 |
| 9 | >10000 | 890.5 |
| 10 | 83325 | 563.6 |
| 11 | 9356 | 1342 |
| 12 | 6320 | 796.3 |
| 13 | >10000 | 1543.2 |
| 14 | >10000 | 990.7 |
| 15 | >10000 | 783.9 |
| 16 | >10000 | 7530 |
| 17 | 6329 | 710.3 |
| 18 | 9107 | 3570 |
| 19 | >10000 | 891.2 |
| 20 | >10000 | 997.7 |
| 21 | >10000 | 1356.8 |
| 22 | >10000 | 433.5 |
| 23 | 9832 | 2531.2 |
| 24 | >10000 | 773.5 |
| 25 | >10000 | 1506.3 |
| 26 | >10000 | 3668.4 |
| 27 | >10000 | 7528.1 |
| 28 | >10000 | 1003.8 |
| 29 | >10000 | 866.3 |
| 30 | >10000 | 561.6 |
| 31 | >10000 | 533.2 |
| 32 | >10000 | 1993.8 |
| Clorgyline | 4.2 | |
| Selegiline hydrochloride | | 119.4 |

The foregoing descriptions are merely specific embodiments of the present invention, but the protection scope of the present invention is not limited thereto. Any person skilled in the art can easily conceive of variations or substitutions within the technical scope disclosed by the present invention, and all such variations or substitutions shall fall within the protection scope of the present invention. Therefore, the protection scope of the present invention shall be subject to the protection scope of the claims.

## Claims

1. A biphenyltetrahydropyrrole compound of Formula I, or an isotopically labeled compound thereof, or an optical isomer, geometric isomer, tautomer or isomer mixture thereof, or a pharmaceutically acceptable salt thereof, or a prodrug thereof, or a metabolite thereof: 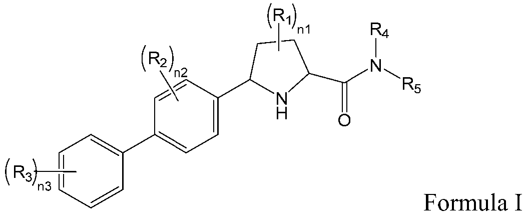 wherein,
R₁ is selected from the group consisting of hydrogen, deuterium, halogen, cyano, hydroxy, amino, amido, sulfonyl, C₁-C₆ alkyl, C₃-C₆ cycloalkyl, C₁-C₆ alkoxy, and C₃-C₆ cycloalkoxy;
R₂ is selected from the group consisting of hydrogen, deuterium, halogen, cyano, hydroxy, amino, amido, sulfonyl, C₁-C₆ alkyl, C₃-C₆ cycloalkyl, C₁-C₆ alkoxy, and C₃-C₆ cycloalkoxy;
R₃ is selected from the group consisting of hydrogen, deuterium, halogen, cyano, hydroxy, nitro, carboxy, amino, amido, sulfonyl, C₁-C₆ alkyl, C₁-C₆ haloalkyl, C₃-C₆ cycloalkyl, C₁-C₆ alkoxy, C₁-C₆ haloalkoxy, C₃-C₆ cycloalkoxy, -C(=O)OC₁-C₆ alkyl, and -C(=O)ONH₂;
R₄ and R₅ are each independently selected from the group consisting of hydrogen, halogen, cyano, amido, sulfonyl, C₁-C₆ alkyl, C₃-C₆ cycloalkyl, C₁-C₆ alkoxy, and C₃-C₆ cycloalkoxy;
n1 is an integer selected from 0 to 2;
n2 is an integer selected from 0 to 4;
n3 is an integer selected from 0 to 5.

2. The biphenyltetrahydropyrrole compound of Formula I according to claim 1, or an isotopically labeled compound thereof, or an optical isomer, geometric isomer, tautomer or isomer mixture thereof, or a pharmaceutically acceptable salt thereof, or a prodrug thereof, or a metabolite thereof, wherein,
preferably, R₁ is selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy;
preferably, R₁ is selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexoxy;
preferably, R₁ is selected from the group consisting of hydrogen, deuterium, halogen, cyano, and hydroxy;
preferably, R₂ is selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy;
preferably, R₂ is selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexoxy;
preferably, R₂ is selected from the group consisting of hydrogen, deuterium, halogen, cyano, and hydroxy;
R₃ is selected from the group consisting of hydrogen, deuterium, halogen, cyano, hydroxy, nitro, carboxy, amino, amido, sulfonyl, C₁-C₄ alkyl, C₁-C₄ haloalkyl, C₃-C₆ cycloalkyl, C₁-C₄ alkoxy, C₁-C₄ haloalkoxy, C₃-C₆ cycloalkoxy, -C(=O)OC₁-C₄ alkyl, and -C(=O)ONH₂;
preferably, R₃ is selected from the group consisting of hydrogen, deuterium, halogen, cyano, hydroxy, nitro, carboxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, cyclohexoxy, monofluoromethyl, difluoromethyl, trifluoromethyl, dichloromethyl, trichloromethyl, monofluoroethyl, difluoroethyl, trifluoroethyl, tetrafluoroethyl, pentafluoroethyl, dichloroethyl, trichloroethyl, tetrachloroethyl, pentachloroethyl, difluoropropyl, trifluoropropyl, tetrafluoropropyl, pentafluoropropyl, hexafluoropropyl, perfluoropropyl, monochloropropyl, dichloropropyl, trichloropropyl, tetrachloropropyl, pentachloropropyl, hexachloropropyl, perchloropropyl, monofluoromethoxy, difluoromethoxy, trifluoromethoxy, dichloromethoxy, trichloromethoxy, monofluoroethoxy, difluoroethoxy, trifluoroethoxy, tetrafluoroethoxy, pentafluoroethoxy, dichloroethoxy, trichloroethoxy, tetrachloroethoxy, pentachloroethoxy, difluoropropoxy, trifluoropropoxy, tetrafluoropropoxy, pentafluoropropoxy, hexafluoropropoxy, perfluoropropoxy, monochloropropoxy, dichloropropoxy, trichloropropoxy, tetrachloropropoxy, pentachloropropoxy, hexachloropropoxy, perchloropropoxy, -C(=O)O methyl, -C(=O)O ethyl, -C(=O)O n-propyl, -C(=O)O isopropyl, -C(=O)O n-butyl, -C(=O)O sec-butyl, -C(=O)O tert-butyl, and -C(=O)ONH₂;
preferably, R₃ is selected from the group consisting of hydrogen, deuterium, halogen, cyano, hydroxy, nitro, carboxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, trifluoromethyl, -C(=O)O methyl, -C(=O)O ethyl, -C(=O)O n-propyl, -C(=O)O isopropyl, -C(=O)O n-butyl, -C(=O)O sec-butyl, -C(=O)O tert-butyl, and -C(=O)ONH₂;
preferably, R₄ and R₅ are each independently selected from the group consisting of hydrogen, deuterium, halogen, cyano, hydroxy, amino, amido, sulfonyl, C₁-C₄ alkyl, C₃-C₆ cycloalkyl, C₁-C₄ alkoxy, and C₃-C₆ cycloalkoxy;
preferably, R₄ and R₅ are each independently selected from the group consisting of hydrogen, deuterium, halogen, cyano, hydroxy, amino, amido, sulfonyl, methyl, ethyl, n-propyl, isopropyl, n-butyl, sec-butyl, tert-butyl, cyclopropyl, cyclobutyl, cyclopentyl, cyclohexyl, methoxy, ethoxy, n-propoxy, isopropoxy, n-butoxy, sec-butoxy, tert-butoxy, cyclopropoxy, cyclobutoxy, cyclopentoxy, and cyclohexoxy;
preferably, R₄ and R₅ are each independently selected from the group consisting of hydrogen, deuterium, halogen, cyano, and hydroxy;
preferably, n1 is an integer selected from 0, 1, or 2;
preferably, n2 is an integer selected from 0, 1, 2, or 3;
preferably, n3 is an integer selected from 0, 1, 2, or 3.

3. The biphenyltetrahydropyrrole compound of Formula I according to claim 1, or an isotopically labeled compound thereof, or an optical isomer, geometric isomer, tautomer or isomer mixture thereof, or a pharmaceutically acceptable salt thereof, or a prodrug thereof, or a metabolite thereof, wherein,
preferably, the compound of Formula I, or an isotopically labeled compound thereof, or an optical isomer, geometric isomer, tautomer or mixture of isomers thereof, or a pharmaceutically acceptable salt thereof, or a prodrug thereof, or a metabolite thereof is represented by the following Formula II: 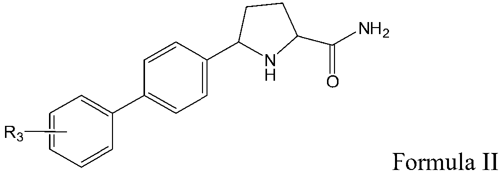
wherein R₃ is defined the same as that in claim 1.

4. The biphenyltetrahydropyrrole compound of Formula I or II according to any one of claims 1 to 3, or an isotopically labeled compound thereof, or an optical isomer, geometric isomer, tautomer or isomer mixture thereof, or a pharmaceutically acceptable salt thereof, or a prodrug thereof, or a metabolite thereof, wherein the compound is selected from the group consisting of the following compounds:

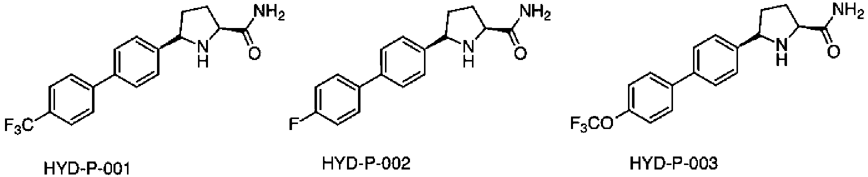

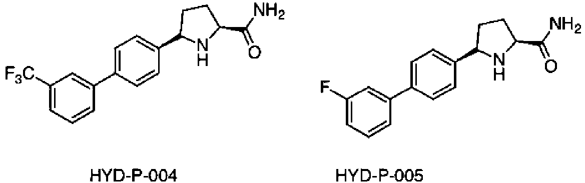

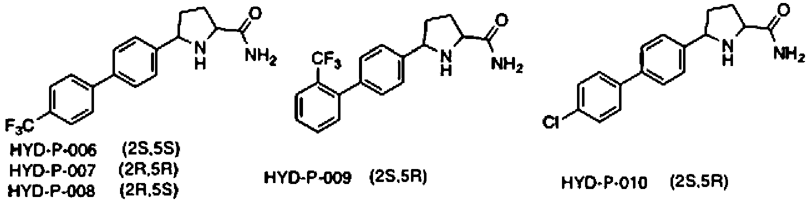

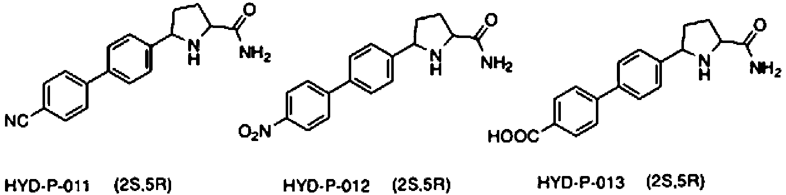

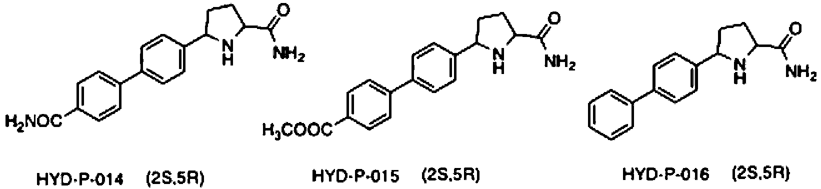

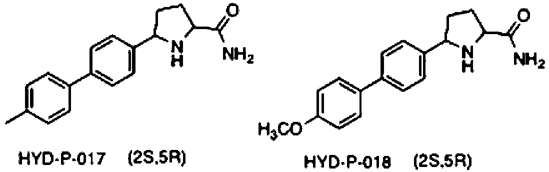

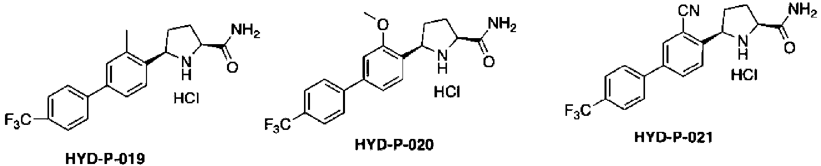

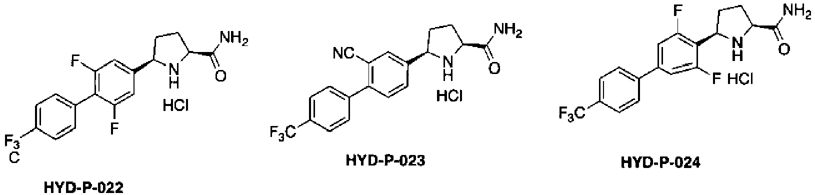

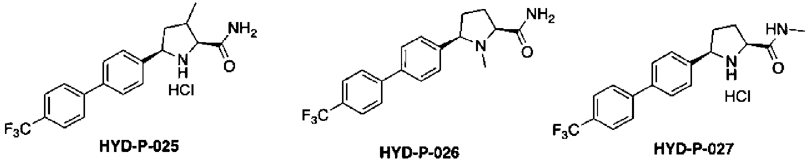

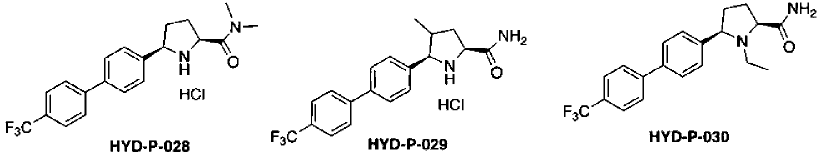

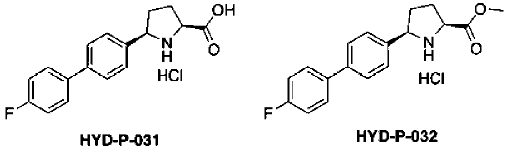

5. A preparation method for the biphenyltetrahydropyrrole compound of Formula I or II according to any one of claims 1 to 4, or an isotopically labeled compound thereof, or an optical isomer, geometric isomer, tautomer or isomer mixture thereof, or a pharmaceutically acceptable salt thereof, or a prodrug thereof, or a metabolite thereof, wherein the preparation method is carried out as follows: 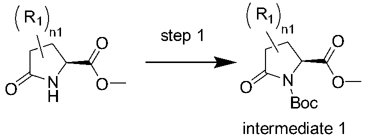 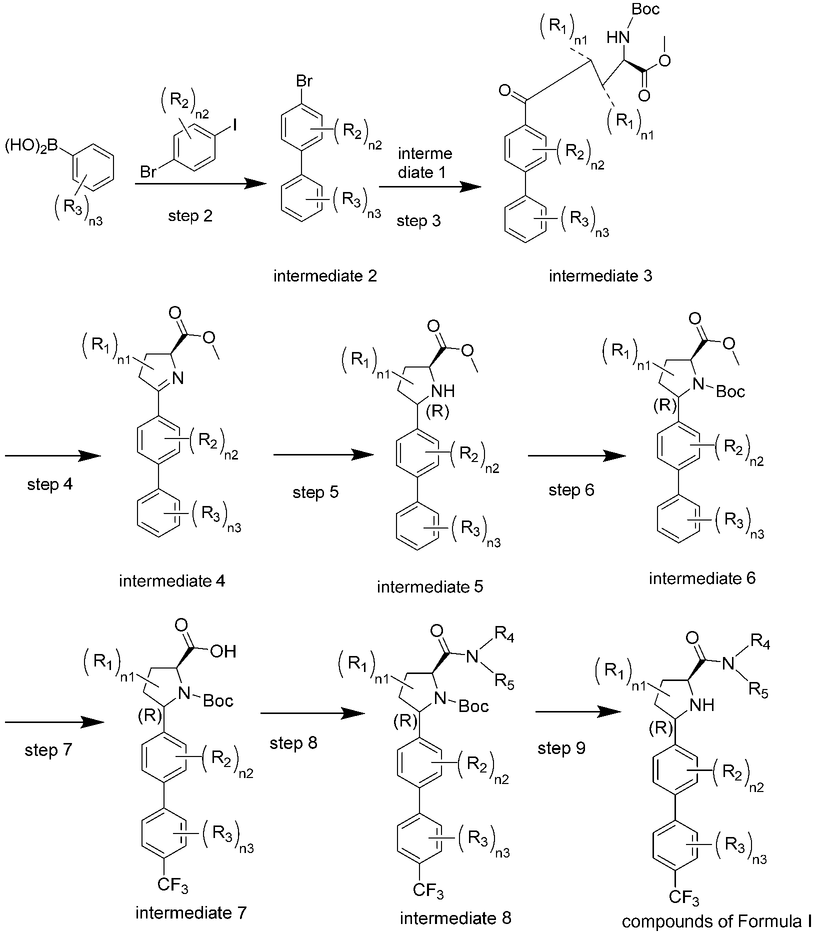
1) a methyl pyroglutamate containing a substituent R₁ is added to an organic solvent (dichloromethane, chloroform, tetrahydrofuran, etc.), and at room temperature, di-tert-butyl decarbonate (Boc₂O) and N,N-dimethylaminopyridine (DMAP) are added to generate tert-Butoxycarbonyl-protected methyl L-pyroglutamate containing the substituent R₁, as **intermediate 1;**
2) a phenylboronic acid containing a substituent R₃ and a p-bromoiodobenzene containing a substituent R₂ with different groups are added at 25°C with sodium carbonate (Na₂CO₃) and bis(triphenylphosphine)palladium (II) chloride [Pd(PPh₃)₂Cl₂], and stirred at 90°C for 2 hours to obtain a brominated biphenyl compound as **intermediate 2;**
3) under nitrogen atmosphere, **intermediate 2** dissolved in tetrahydrofuran is added dropwise with n-butyllithium at-78°C, and stirred for 1 to 2 hours while maintaining the temperature, a solution of **intermediate** 1 in dry tetrahydrofuran is added dropwise at -78°C, and the mixture is reacted for 40 minutes while maintaining the temperature, then raised to -40°C and reacted for 2 to 3 hours to obtain **intermediate 3;**
4) **intermediate 3** is dissolved in dichloromethane at room temperature, added with trifluoroacetic acid, and reacted overnight to obtain **intermediate 4;**
5) **intermediate 4** is dissolved in methanol at room temperature under nitrogen atmosphere, added with platinum(IV) oxide, and reacted overnight to obtain **intermediate 5;**
6) **intermediate 5** is dissolved in dichloromethane at room temperature, and added with di-tert-butyl dicarbonate to obtain **intermediate 6** whose secondary amine is protected;
7) **intermediate 6** is dissolved in tetrahydrofuran and water at room temperature, added with lithium hydroxide, raised to 50°C, and stirred for 3 to 4 hours to obtain a carboxylic acid compound as **intermediate 7;**
8) **intermediate 7** is dissolved in N,N-dimethylformamide at room temperature, added with an amine reagent containing R₄ and R₃ groups, 2-(7-azabenzotriazol-1-yl)-N,N,N',N'-tetramethyluronium hexafluorophosphate and N,N-diisopropylethylamine, and reacted at room temperature for 3 to 5 hours to obtain an amide compound as **intermediate 8;**
9) **intermediate 8** is dissolved in dichloromethane at room temperature, added with a solution of hydrogen chloride in ethyl acetate, stirred for 2 to 4 hours, and concentrated, the residue is added with water and adjusted to pH 7-8 with a 5% aqueous sodium bicarbonate solution to obtain the compound of Formula 1;
wherein, the dashed line in **intermediate 3** indicates that the substituent R₁ may be present or absent.

6. A pharmaceutical composition comprising a therapeutically effective amount of the biphenyltetrahydropyrrole compound of Formula I or II according to any one of claims 1 to 4, or an isotopically labeled compound thereof, or an optical isomer, geometric isomer, tautomer or isomer mixture thereof, or a pharmaceutically acceptable salt thereof, or a prodrug thereof, or a metabolite thereof, and a pharmaceutically acceptable carrier.

7. Use of the biphenyltetrahydropyrrole compound of Formula I or II according to any one of claims 1 to 4, or anisotopically labeled compound thereof, or an optical isomer, geometric isomer, tautomer or isomer mixture thereof, or a pharmaceutically acceptable salt thereof, or a prodru thereof g, or a metabolite thereof, in the preparation of a medicament for a Monoamine Oxidase B inhibitor, the medicament is used for treating or preventing a disease or condition associated with Monoamine Oxidase B in a subject in need thereof;
preferably, the disease or condition associated with Monoamine Oxidase B is a neuropsychiatric disease or condition;
more preferably, the neuropsychiatric disease or condition is selected from the group consisting of Depression, Alzheimer's Disease, Parkinson's Disease, Senile Dementia, and Narcolepsy.

8. A kit comprising the biphenyltetrahydropyrrole compound of Formula I or II according to any one of claims 1 to 4, or an isotopically labeled compound thereof, or an optical isomer, geometric isomer, tautomer or isomer mixture thereof, or a pharmaceutically acceptable salt thereof, or a prodrug thereof, or a metabolite thereof, or the pharmaceutical composition according to claim 6, and a container and an instruction for use.

9. A method for treating a disease or condition associated with Monoamine Oxidase B, comprising administering to a subject in need thereof an effective amount of the biphenyltetrahydropyrrole compound of Formula I or II according to any one of claims 1 to 4, or an isotopically labeled compound thereof, or an optical isomer, geometric isomer, tautomer or isomer mixture thereof, or a pharmaceutically acceptable salt thereof, or a prodrug thereof, or a metabolite thereof, or the pharmaceutical composition according to claim 6.
